# EUROPEAN PATENT APPLICATION

(11) **EP 4 729 082 A1**
(43) Date of publication of application: **22.04.2026**
(21) Application number: 24823382.7
(22) Date of filing: 11.06.2024
(51) Int. Cl.: A61M 1/00

(54) **MEDICAL SYSTEM AND LIQUID CIRCULATION SYSTEM**

(30) Priority: 16.06.2023 JP 2023099693; 11.09.2023 JP 2023147225
(71) Applicant: Terumo Kabushiki Kaisha, Tokyo 151-0072 (JP)
(72) Inventor: KITAOKA, Takashi, Ashigarakami-gun Kanagawa 259-0151 (JP)
(74) Representative: KIPA AB
(86) International application number: PCT/JP2024/021221
(87) International publication number: WO 2024/257767

(57) **Abstract**

An object is to provide the medical system and the liquid circulation system capable of efficiently delivering the liquid to the treatment area of the brain while suppressing the fluctuations in the intracranial pressure. A medical system includes a control unit that executes control of setting a first period 211 in which the liquid is injected into the body cavity and the liquid is not discharged from the body cavity and a second period 212 in which the liquid is not injected into the body cavity and the liquid is discharged from the body cavity, and alternately repeating the first period 211 and the second period 212 to set an injection amount of the liquid in the first period 211 to be substantially the same as a discharge amount of the liquid in the second period 212.

## Description

### Technical Field

The present invention relates to a medical system and a liquid circulation system which are used for treatment of a brain disease.

### Background Art

Development of a brain disease such as cerebral infarction blocks blood flow for supplying oxygen to brain cells, which may cause a risk of damaging the brain cells. Therefore, when the cerebral infarction occurs, early reperfusion of the blood flow is necessary. As one of treatments for the cerebral infarction, it has been proposed that a high oxygen solution such as oxygenated cerebrospinal fluid is injected into a body cavity in which cerebrospinal fluid of a patient is present, and oxygen is directly supplied to oxygen-deficient brain cells.

Patent Literature 1 discloses a catheter system capable of introducing and drawing a liquid into and from a living body through a single lumen. The catheter system described in Patent Literature 1 includes a catheter, an injection actuator for supplying a fluid to an injection port through a lumen in a tubular body, a suction actuator for drawing in a fluid from a suction port through the lumen in the tubular body, a detector for detecting a state of intracranial pressure, and a control unit for controlling the injection actuator and the suction actuator. The suction port of the catheter is in a closed state when the injection port of the catheter is in an open state. In addition, the injection port of the catheter is in a closed state when the suction port of the catheter is in an open state. Then, the control unit alternately operates the injection actuator and the suction actuator in order to keep the state of intracranial pressure detected by the detector within a certain range.

Here, in a case where a liquid such as a high oxygen solution is injected into a body cavity for the purpose of treating a brain disease, it is necessary to keep the intracranial pressure within a certain range. A normal range of the intracranial pressure is about 5 mmHg to 15 mmHg. If the pressure from an intracranial cavity to an intraspinal cavity fluctuates, there is a possibility that a brain is damaged. For example, when the intracranial pressure increases to 20 mmHg or more, there is a possibility that intracranial hypertension (that is, various symptoms developed by a load on brain tissue) occurs. On the other hand, when the intracranial pressure decreases to 5 mmHg or less, there is a possibility that cerebrospinal fluid hypovolemia (that is, various symptoms developed as a position of the brain tissue cannot be kept) occurs.

The body cavity (for example, a subarachnoid space and cerebral ventricles) in which cerebrospinal fluid is present is a substantially closed space. Therefore, in order to keep the intracranial pressure within the certain range, it is necessary to keep the amount of the cerebrospinal fluid in the body cavity within a certain range. In order to keep the amount of the cerebrospinal fluid in the body cavity within the certain range, it is required to rapidly discharge the same amount of cerebrospinal fluid as the amount of the liquid injected into the body cavity.

However, for example, in a case where a liquid is circulated by a pump through a catheter for injection and a catheter for discharge, which are inserted into the subarachnoid space, from the vicinity of lumbar vertebrae to inject the liquid into the body cavity, a flow (that is, locally steady flow) in which the liquid injected into the body cavity from an injection port of the catheter for injection flows toward a discharge port of the catheter for discharge is formed depending on an injection timing and a discharge timing. The locally steady flow decreases a flow of the liquid from the injection port of the catheter for injection toward a treatment area of the brain, and there is a problem that the liquid cannot be efficiently delivered to the treatment area of the brain.

In order to solve this problem, it is conceivable to dispose the injection port of the catheter for injection in the vicinity of the brain and dispose the discharge port of the catheter for discharge in the vicinity of the lumbar vertebrae, thereby sufficiently securing a distance between the injection port and the discharge port and suppressing an influence of the locally steady flow as much as possible. However, in a case where an attempt is made to insert the catheter into the vicinity of the brain, the catheter is inserted over a relatively long distance into the curved subarachnoid space around which nerve tissue such as the spinal cord runs. Since such insertion of the catheter into a deep portion of the subarachnoid space involves a risk of damaging the nerve tissue, it is desirable to shorten an insertion length of the catheter as much as possible.

In addition, for example, in a case where a liquid is circulated by a pump through a catheter for injection and a catheter for discharge inserted into the subarachnoid space from the vicinity of the lumbar vertebrae to inject the liquid into the body cavity, in order to keep the amount of cerebrospinal fluid in the body cavity within a certain range, it is necessary to control parameters such as the cross-sectional area of the lumen, the fluid resistance due to the surface property of the lumen, and the length of each of the catheter for injection and the catheter for discharge, and to make the injection amount and the discharge amount constant. However, variations in the above-described parameters occur due to variations in manufacturing the catheter and deformation of the cross-sectional shape of the lumen of the catheter at the time of use. Therefore, it is difficult to make the injection amount and the discharge amount constant.

In addition, a circulation circuit of the liquid is desirably a closed system for infection prevention. However, in a case where the circulation circuit of the liquid is the closed system, there is a possibility that air bubbles are generated in the circulation circuit caused by the cavitation phenomenon to increase a total volume of the liquid from a volume in an initial stage of a treatment, or water is lost caused by evaporation in the process of oxygenating the cerebrospinal fluid to decrease the total volume of the liquid from the volume in the initial stage of the treatment. If the total volume of the liquid increases or decreases in the circulation circuit of the closed system, there is a possibility that the intracranial pressure cannot be kept in the certain range.

### Citation List

### Patent Literature

Patent Literature 1: WO 2021/192539 A1

### Summary of Invention

### Technical Problem

The present invention has been made in view of the above circumstances, and an object thereof is to provide a medical system and a liquid circulation system capable of efficiently delivering a liquid to a treatment area of a brain while suppressing fluctuations in intracranial pressure.

### Solution to Problem

The present invention provides (1) a medical system that injects a liquid into a body cavity in which cerebrospinal fluid of a subject is present and discharges the liquid present in the body cavity from the body cavity, the medical system comprising a control unit that executes control of setting a first period in which the liquid is injected into the body cavity and the liquid is not discharged from the body cavity and a second period in which the liquid is not injected into the body cavity and the liquid is discharged from the body cavity, and alternately repeating the first period and the second period to set an injection amount of the liquid in the first period to be substantially the same as a discharge amount of the liquid in the second period.

According to the medical system of (1), the control unit injects the liquid into the body cavity and does not discharge the liquid from the body cavity in the first period. In addition, the control unit does not inject the liquid into the body cavity and discharges the liquid from the body cavity in the second period. Then, the control unit alternately repeats the first period and the second period to make an injection amount of the liquid substantially identical to a discharge amount of the liquid. As a result, the injection of the liquid and the discharge of the liquid are alternately repeated in the same amount as long as an allowable fluctuation range of the intracranial pressure is not exceeded. Therefore, the injection of the liquid and the discharge of the liquid are not simultaneously performed, and it is possible to suppress the generation of the locally steady flow in which the liquid injected into the body cavity from the injection port flows toward the discharge port. Therefore, the liquid injected into the body cavity flows toward the treatment area of the brain according to the injection direction and the injection flow rate. In addition, the liquid and the cerebrospinal fluid are stirred by the turbulence generated by the resistance between the liquid injected into the body cavity and the cerebrospinal fluid in the body cavity. Furthermore, diffusion due to a concentration difference of oxygen or the like contained in the liquid proceeds. As a result, the liquid can be efficiently delivered to a treatment area of a brain. In addition, since the injection amount of the liquid is substantially the same as the discharge amount of the liquid, the amount of cerebrospinal fluid in the body cavity can be kept within a certain range. This can suppress substantial fluctuations in intracranial pressure.

(2) In the medical system of (1), it is preferable that a cycle from the start of the first period to the end of the second period is substantially constant.

According to the medical system of (2), at least one of stirring of the liquid and the cerebrospinal fluid and diffusion due to a concentration difference of oxygen or the like contained in the liquid stably progresses. As a result, the liquid can be efficiently delivered to a treatment area of a brain. In addition, the amount of the cerebrospinal fluid in the body cavity can be stably kept in the certain range. This can suppress fluctuations in intracranial pressure.

(3) In the medical system of (1) or (2), it is preferable that the control unit further sets, between the first period and the second period, a third period in which the liquid is not injected into the body cavity and the liquid is not discharged from the body cavity.

According to the medical system of (3), the diffusion due to the concentration difference of oxygen or the like contained in the liquid further progresses according to the length of the third period. As a result, the liquid can be more efficiently delivered to a treatment area of a brain.

(4) In the medical system of (3), the duration of the third period is preferably longer than the duration of the first period.

According to the medical system of (4), the time during which diffusion due to the concentration difference of oxygen or the like contained in the liquid progresses is longer than the injection time of the liquid. As a result, the liquid can be more efficiently delivered to a treatment area of a brain.

(5) In the medical system of (3) or (4), it is preferable that the duration of the third period is 1 second or more.

According to the medical system of (5), it is possible to secure a time during which diffusion due to a concentration difference of oxygen or the like contained in the liquid progresses. As a result, the liquid can be efficiently delivered to a treatment area of a brain.

(6) In the medical system of any one of (3) to (6), the control unit preferably further sets the third period between the second period and the first period, and a first time of the third period set between the first period and the second period is longer than a second time of the third period set between the second period and the first period.

According to the medical system of (6), the first time of the stop period (that is, third period) provided between the injection of the liquid and the discharge of the liquid is longer than the second time of the stop period (that is, third period) provided between the discharge of the liquid and the injection of the liquid. Therefore, it is possible to more reliably suppress the generation of the locally steady flow in which the liquid injected into the body cavity from the injection port flows toward the discharge port, and it is possible to reliably secure the time during which the diffusion due to the concentration difference of oxygen or the like contained in the liquid progresses. As a result, the liquid can be more efficiently delivered to a treatment area of a brain.

(7) In the medical system of any one of (1) to (6), it is preferable that a discharge rate of the liquid in the second period is slower than an injection rate of the liquid in the first period.

According to the medical system of (7), since the discharge rate of the liquid in the second period is slower than the injection rate of the liquid in the first period, it is possible to suppress the rate at which the liquid injected into the body cavity from the injection port flows toward the discharge port, and it is possible to secure the time during which the liquid immediately after being injected into the body cavity from the injection port diffuses in the direction of the brain. As a result, the liquid can be efficiently delivered to a treatment area of a brain.

(8) In the medical system of any one of (1) to (7), it is preferable that the control unit sets a 0th period for injecting or discharging an amount of the liquid smaller than the injection amount in the first period and the discharge amount in the second period as an initial period, and starts a period, among the first period and the second period, in which a moving direction of the liquid is different from that in the 0th period after the 0th period.

According to the medical system of (8), by reducing the change in the capacity in the subarachnoid space in the initial 0th period, the change in the capacity in the subarachnoid space in the first period and the second period after the 0th period varies within a range encompassing the original capacity. Therefore, it is possible to suppress exceeding the allowable fluctuation range. This can suppress fluctuations in intracranial pressure within the allowable fluctuation range.

(9) In the medical system of (8), it is preferable that an injection amount in the 0th period is substantially half of the injection amount in the first period, and a discharge amount in the 0th period is substantially half of the discharge amount in the second period.

According to the medical system of (9), it is possible to more reliably suppress the intracranial pressure from exceeding the allowable fluctuation range due to the injection of the liquid in the initial first period. This makes it possible to more reliably suppress the fluctuations in the intracranial pressure within the allowable fluctuation range.

(10) In the medical system of any one of (1) to (9), it is preferable that at the end of the control, the control unit performs control to cause an amount of the cerebrospinal fluid to approach an amount of the cerebrospinal fluid existing in the body cavity at the start of execution of the control by injecting the liquid into the body cavity or to cause an amount of the cerebrospinal fluid to approach an amount of the cerebrospinal fluid existing in the body cavity at the start of execution of the control by discharging the liquid from the body cavity.

According to the medical system of (10), the amount of cerebrospinal fluid at the end of the control of the medical system can be set to be substantially the same as the amount of cerebrospinal fluid existing in the body cavity at the start of the execution of the control of the medical system. Therefore, the intracranial pressure at the end of the control of the medical system can be set to be substantially the same as the intracranial pressure at the start of execution of the control of the medical system. As a result, it is possible to suppress the fluctuation in the intracranial pressure between the end of the control of the medical system and the start of execution of the control of the medical system.

(11) It is preferable that the medical system of any one of (1) to (10) further includes: an injection catheter that injects the liquid into the body cavity; and a discharge catheter that discharges the liquid from the body cavity, in which a distance between a distal end of the injection catheter and a distal end of the discharge catheter is 30 cm or less at a start of execution of the control.

According to the medical system of (11), since the injection of the liquid and the discharge of the liquid are alternately repeated in the same amount, it is possible to suppress the generation of the locally steady flow in which the liquid injected into the body cavity from the injection port flows toward the discharge port even when the distance between the distal end of the injection catheter and the distal end of the discharge catheter is 30 cm or less at the start of execution of the control. As a result, the liquid can be efficiently delivered to the treatment area of the brain without deeply inserting the injection catheter into a living body.

(12) In the medical system of (1), it is preferable that the control unit further executes control to deflate a balloon disposed in the body cavity and capable of inflation and deflation in the first period and to inflate the balloon in the second period.

According to the medical system of (12), in the first period, the control unit injects the liquid into the body cavity, does not discharge the liquid from the body cavity, and further deflates the balloon disposed in the body cavity by the same volume with the liquid. As a result, the control unit can suppress the fluctuation in the volume of the body cavity as the closed space in the first period in which the liquid is injected into the body cavity, and thus, can suppress the fluctuation in the intracranial pressure. In addition, in the second period, the control unit does not inject the liquid into the body cavity and discharges the liquid from the body cavity, and further inflates the balloon disposed in the body cavity by the same volume as the liquid. As a result, the control unit can suppress the fluctuation in the volume of the body cavity as the closed space in the second period in which the liquid is discharged from the body cavity, and thus, can suppress the fluctuation in the intracranial pressure.

In the first period and the second period, there is a case where slight volume fluctuations occur due to a difference between the liquid discharge amount and the balloon inflation amount, a difference between the liquid injection amount and the balloon deflation amount, or the like. The volume fluctuation amount in this case is the volume fluctuation amount within the volume change capability of the body cavity of the subject, that is, the adaptive (compliance) allowable range for maintaining the body cavity internal pressure. Therefore, when the living body is deformed following the decreased or increased amount of liquid, the volume fluctuation can be canceled while the intracranial pressure is maintained constant.

In the medical system of (12), it is also possible to supplementarily use the inflation and deflation of the balloon. By inflating and deflating the balloon with a volume smaller than the amount of the delivered liquid, the volume fluctuation in the body cavity is partially canceled, and the volume fluctuation amount is suppressed to fall within an area that can be handled by the compliance of the living body. This allows for delivery with a larger volume of liquid beyond the high compliance area, as well as providing the same effect as when the balloon inflates and deflates by the same volume with the volume of liquid delivered.

(13) In the medical system of (12), it is preferable that the control unit simultaneously executes the injection of the liquid and the deflation of the balloon in the first period, and simultaneously executes the discharge of the liquid and the inflation of the balloon in the second period.

According to the medical system of (13), the control unit simultaneously executes the injection of the liquid and the deflation of the balloon in the first period. The control unit can further suppress the fluctuation in the volume of the body cavity in the first period in which the liquid is injected into the body cavity. Therefore, the fluctuation in the intracranial pressure in the first period is further suppressed. The control unit simultaneously executes discharge of the liquid and inflation of the balloon in the second period. Therefore, the control unit can further suppress the fluctuation in the volume of the body cavity in the second period in which the liquid is discharged from the body cavity. Therefore, the fluctuation in the intracranial pressure in the second period is further suppressed.

(14) In the medical system of (12) or (13), it is preferable that the control unit sets a deflation amount of the balloon to be substantially the same as an injection amount of the liquid in the first period, and sets an inflation amount of the balloon to be substantially the same as a discharge amount of the liquid in the second period.

According to the medical system of (14), the control unit sets the deflation amount of the balloon to be substantially the same as the injection amount of the liquid in the first period. As such, the amount (that is, volume) of liquid increased in the body cavity is substantially the same as the amount (that is, volume) of balloon deflated in the body cavity. Therefore, the volume of the body cavity does not substantially change in the first period. As a result, the fluctuation in the intracranial pressure in the first period is more reliably suppressed. In addition, the control unit sets the inflation amount of the balloon to be substantially the same as the discharge amount of the liquid in the second period. As such, the amount (that is, volume) of liquid reduced in the body cavity is substantially the same as the amount (that is, volume) of balloon inflated in the body cavity. Therefore, the volume of the body cavity does not substantially change in the second period. As a result, the fluctuation in the intracranial pressure in the second period is more reliably suppressed.

(15) In the medical system of any one of (12) to (14), it is preferable that the control unit further sets a third period in which the liquid is not injected into the body cavity and the liquid is not discharged from the body cavity between the first period and the second period and between the second period and the first period, maintains the balloon in the deflated state in the third period between the first period and the second period, and maintains the balloon in the inflated state in the third period between the second period and the first period.

According to the medical system of (15), the diffusion due to the concentration difference of oxygen or the like contained in the liquid further progresses according to the length of the third period. As a result, the liquid can be more efficiently delivered to a treatment area of a brain.

(16) The present invention is a liquid circulation system that circulates a liquid by injecting the liquid into a body cavity in which cerebrospinal fluid is present and discharging the liquid to the outside of the body cavity, the liquid circulation system including: an injection catheter that injects the liquid into the body cavity; a discharge catheter that discharges the liquid from the inside of the body cavity to the outside of the body cavity; a liquid delivery line connected to the injection catheter; a liquid discharge line connected to the discharge catheter; a first liquid delivery unit that is provided on the liquid delivery line and moves the liquid; a second liquid delivery unit that is provided on the liquid discharge line and moves the liquid; and a single control unit that controls the first liquid delivery unit and the second liquid delivery unit.

According to the liquid circulation system of (16), a circuit (that is, biological circuit unit) including the injection catheter and the discharge catheter and a circuit (that is, system circuit unit) including the liquid delivery line connected to the injection catheter and the liquid discharge line connected to the discharge catheter are divided. In addition, a first liquid delivery unit that is provided on the liquid delivery line and moves liquid and a second liquid delivery unit that is provided on the liquid discharge line and moves liquid function as a common liquid delivery unit in the biological circuit and the system circuit. Since the liquid circulation system has such a configuration, the system circuit can cope with an increase in a total volume of the liquid caused by the cavitation phenomenon and a decrease in the total volume of the liquid caused by evaporation while maintaining a situation on the living body side, and increase and decrease in the total volume of the liquid in the biological circuit can be minimized. This can suppress fluctuations in intracranial pressure.

(17) The liquid circulation system of (16) preferably further includes: a liquid storage unit that stores at least one of the liquid to be injected into the body cavity and the liquid discharged from the body cavity; and a liquid treatment device provided between the liquid storage unit in the liquid delivery line and the first liquid delivery unit to treat the liquid.

(18) In the liquid circulation system of (16), it is preferable that the liquid treatment device is an oxygenator that adds oxygen to the liquid discharged out of the body cavity.

According to the liquid circulation system of (18), it is possible to efficiently deliver the high oxygen solution to the treatment area of the brain while suppressing the fluctuations in the intracranial pressure.

(19) In the liquid circulation system of any one of (16) to (18), the control unit preferably alternately and repeatedly drives the first liquid delivery unit and the second liquid delivery unit.

According to the liquid circulation system of (19), the injection of the liquid and the discharge of the liquid are alternately repeated in the same amount as long as an allowable fluctuation range of the intracranial pressure is not exceeded. Therefore, the injection of the liquid and the discharge of the liquid are not simultaneously performed, and it is possible to suppress the generation of the locally steady flow generated when the injection of the liquid and the discharge of the liquid are simultaneously performed, that is, the generation of the locally steady flow in which the liquid injected into the body cavity from the injection port flows toward the discharge port. Therefore, the liquid injected into the body cavity flows toward the treatment area of the brain according to the injection direction and the injection flow rate. In addition, the liquid and the cerebrospinal fluid are stirred by the turbulence generated by the resistance between the liquid injected into the body cavity and the cerebrospinal fluid in the body cavity. Furthermore, diffusion due to a concentration difference of oxygen or the like contained in the liquid proceeds. As a result, the liquid can be efficiently delivered to a treatment area of a brain.

(20) In the liquid circulation system of any one of (16) to (19), the control unit preferably controls a sum of an injection amount of the liquid ejected while the first liquid delivery unit is continuously or intermittently activated without interposing the activation of the second liquid delivery unit and a discharge amount of the liquid ejected while the second liquid delivery unit is continuously or intermittently activated without interposing the activation of the first liquid delivery unit from a time of initial activation within a certain range.

According to the liquid circulation system of (20), it is possible to suppress the fluctuation in the amount of cerebrospinal fluid from the time of the initial activation of the first liquid delivery unit and the second liquid delivery unit within a certain range. Therefore, it is possible to more reliably suppress the intracranial pressure from exceeding the allowable fluctuation range. This makes it possible to more reliably suppress the fluctuations in the intracranial pressure within the allowable fluctuation range.

(21) It is preferable that the liquid circulation system of (16) further includes a balloon disposed in the body cavity and capable of inflation and deflation, in which the control unit further controls the inflation and the deflation of the balloon.

According to the liquid circulation system of (21), the control unit controls the operation of the first liquid delivery unit, the operation of the second liquid delivery unit, and the inflation and deflation of the balloon disposed in the body cavity, so that the fluctuation in the volume of the body cavity as the closed space can be suppressed, and the fluctuation in the intracranial pressure can be suppressed.

(22) It is preferable that the liquid circulation system of (21) further includes a balloon drive unit that includes a fluid storage unit for a balloon that stores an inflation fluid for inflating the balloon, and performs an operation of injecting the inflation fluid in the fluid storage unit for a balloon into the balloon and an operation of sending the inflation fluid from the inside of the balloon into the fluid storage unit for a balloon, in which the balloon drive unit injects the inflation fluid into the balloon to inflate the balloon, and discharges the inflation fluid from the balloon to deflate the balloon.

According to the liquid circulation system of (22), a balloon drive unit is further provided. The balloon drive unit includes a fluid storage unit for a balloon that stores an inflation fluid for inflating the balloon, and performs an operation of injecting the inflation fluid in the fluid storage unit for a balloon into the balloon and an operation of discharging the inflation fluid from the inside of the balloon. The balloon drive unit injects an inflation fluid into the balloon to inflate the balloon. Meanwhile, the balloon drive unit discharges the inflation fluid from the balloon to deflate the balloon. This allows the balloon drive unit to individually control inflation and deflation of the balloon. Therefore, the timing and rate of the injection of the liquid and the deflation of the balloon, and the timing and rate of the discharge of the liquid and the inflation of the balloon are set more appropriately. This further suppresses fluctuations in the intracranial pressure.

### Advantageous Effects of Invention

According to the present invention, it is possible to provide the medical system and the liquid circulation system capable of efficiently delivering the liquid to the treatment area of the brain while suppressing the fluctuations in the intracranial pressure.

### Brief Description of Drawings

Fig. 1 is a schematic diagram illustrating an outline of a medical system according to an embodiment of the present invention.
Fig. 2 is a plan view illustrating the vicinity of an injection port of an injection catheter of the present embodiment.
Fig. 3 is a plan view illustrating the vicinity of a discharge port of a discharge catheter of the present embodiment.
Fig. 4 is a cross-sectional view taken along a cross section B-B illustrated in Fig. 3.
Fig. 5 is a schematic view illustrating a flow of a liquid generated in a body cavity immediately after the start of liquid circulation.
Fig. 6 is a schematic view illustrating a flow of the liquid generated in the body cavity during the liquid circulation.
Fig. 7 is a schematic view illustrating a flow of the liquid generated in the body cavity after the stop of the liquid circulation.
Fig. 8 is a graph showing a relationship among an injection amount, a discharge amount, and an intracavitary fluid volume by control of the present embodiment.
Fig. 9 is a schematic view illustrating a flow of the liquid generated in the body cavity by injection in control of the present embodiment.
Fig. 10 is a schematic view illustrating a flow of the liquid generated in the body cavity during the stop of control of the present embodiment.
Fig. 11 is a schematic view illustrating a flow of the liquid generated in the body cavity by discharge in control of the present embodiment.
Fig. 12 is a graph showing a relationship among an injection amount, a discharge amount, and an intracavitary fluid volume of a first modification in control of the present embodiment.
Fig. 13 is a graph showing a relationship among an injection amount, a discharge amount, and an intracavitary fluid volume of a second modification in control of the present embodiment.
Fig. 14 is a graph showing a relationship among an injection amount, a discharge amount, and an intracavitary fluid volume of a third modification in control of the present embodiment.
Fig. 15 is a schematic diagram illustrating an outline of an experiment performed by the present inventor.
Fig. 16 is a table showing an example of results of the experiment performed by the present inventor.
Fig. 17 is a set of photographs showing states of the experiment performed by the present inventor.
Fig. 18 is a schematic diagram illustrating an outline of a liquid circulation system according to the present embodiment.
Fig. 19 is a schematic diagram for describing an operation of the liquid circulation system according to the present embodiment.
Fig. 20 is a schematic diagram for describing an operation of the liquid circulation system according to the present embodiment.
Fig. 21 is a schematic diagram for describing an operation of the liquid circulation system according to the present embodiment.
Fig. 22 is a schematic diagram for describing an operation of the liquid circulation system according to the present embodiment.
Fig. 23 is a schematic diagram illustrating an outline of a liquid circulation system according to the first modification of the present embodiment.
Fig. 24 is a schematic diagram for describing an operation of the liquid circulation system according to the present modification.
Fig. 25 is a schematic diagram for describing an operation of the liquid circulation system according to the present modification.
Fig. 26 is a schematic diagram illustrating an outline of a liquid circulation system according to the second modification of the present embodiment.
Fig. 27 is a schematic diagram illustrating a modification of the medical device of the present embodiment.
Fig. 28 is a graph showing a relationship among an injection amount, a discharge amount, a balloon volume, and an intracavitary fluid volume by control of a medical system according to the second modification of the present embodiment.

### Description of Embodiments

An embodiment of the present invention will be described below with reference to the drawings.

Note that the embodiment described below is a preferred specific example of the present invention, and thus various technically preferable limitations are given. However, the scope of the present invention is not limited to these aspects unless there is a description to limit the present invention in the following description. In addition, in the drawings, similar components are denoted by the same reference signs, and the detailed description thereof will be appropriately omitted.

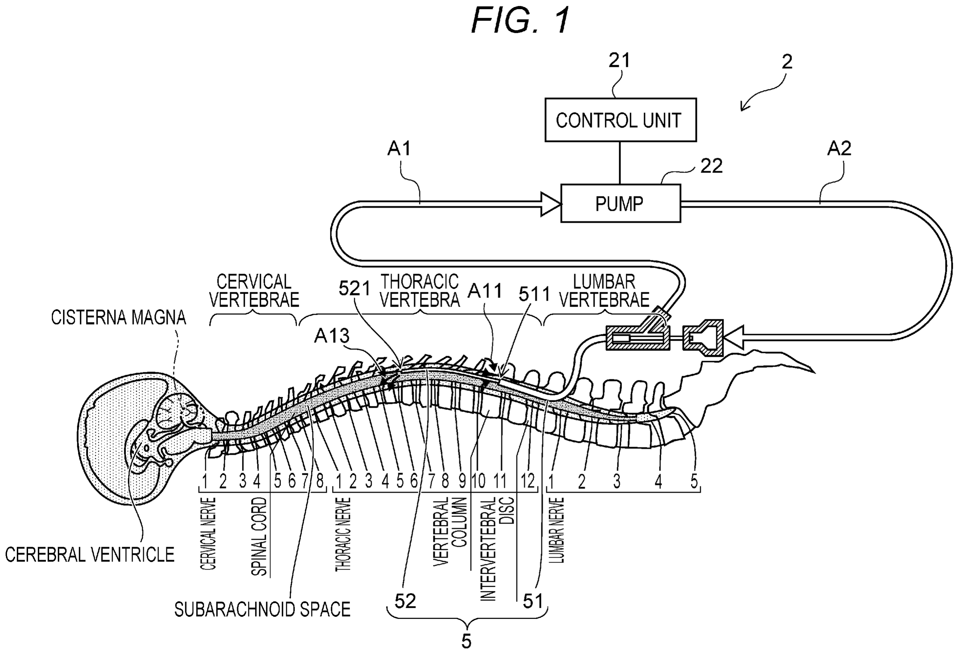

Fig. 1 is a schematic diagram illustrating an outline of a medical system according to the embodiment of the present invention.

A medical system 2 according to the present embodiment injects a liquid into a body cavity in which cerebrospinal fluid (CSF) of a subject is present, and discharges a liquid present in the body cavity from the body cavity. The cerebrospinal fluid is present mainly in a subarachnoid space and cerebral ventricles. That is, the body cavity in which the cerebrospinal fluid is present includes the subarachnoid space and the cerebral ventricles.

As illustrated in Fig. 1, the medical system 2 includes a control unit 21, a pump 22, and a medical device 5. The control unit 21 is connected to the pump 22 and transmits a control signal to the pump 22 to control an operation of the pump 22. The pump 22 operates on the basis of the control signal transmitted from the control unit 21, and draws and discharges the cerebrospinal fluid from the body cavity of the subject through the medical device 5 as indicated by an arrow A1 in Fig. 1. The cerebrospinal fluid discharged out of the body cavity by the pump 22 is generated into a liquid (that is, high oxygen solution) having an oxygen concentration higher than a normal oxygen concentration of cerebrospinal fluid by, for example, an oxygenation mechanism (not illustrated) or the like. Then, the pump 22 injects the liquid into the body cavity through the medical device 5 as indicated by an arrow A2 illustrated in Fig. 1.

Note that the liquid to be injected into the body cavity is not limited to the high oxygen solution. For example, the liquid to be injected into the body cavity may be a liquid containing a drug and obtained by adding the drug to the cerebrospinal fluid during extracorporeal circulation, or may be cerebrospinal fluid filtered with a filter to remove an undesirable substance during extracorporeal circulation. In addition, the liquid to be injected into the body cavity may be one obtained by performing certain processing, such as irradiation with energy or heating, on the cerebrospinal fluid and returned into the body cavity. In the following description, an example in which the liquid to be injected into the body cavity is the high oxygen solution may be given for convenience of description. In addition, in an initial stage of a treatment, lactated Ringer's solution can be used as a substitute for the cerebrospinal fluid in the liquid to be injected into the living body. In the present embodiment, the cerebrospinal fluid and artificial cerebrospinal fluid such as the lactated Ringer's solution, a mixed solution of the cerebrospinal fluid and the lactated Ringer's solution, and the like may be collectively referred to as the liquid.

As illustrated in Fig. 1, the medical device 5 includes a discharge catheter 51 and an injection catheter 52, and is inserted into the subarachnoid space from the vicinity of lumbar vertebrae in a lateral decubitus state. A distal end position where the injection catheter 52 is disposed is desirably between the vicinity of the sixth thoracic vertebra, called T6, from the top and a position of the first lumbar vertebra, called L1, from the top in consideration of safety at the time of insertion. For example, as indicated by an arrow A11 illustrated in Fig. 1, the discharge catheter 51 draws the cerebrospinal fluid present in the body cavity from a discharge port 511 and discharges the cerebrospinal fluid out of the body cavity. For example, as indicated by an arrow A13 illustrated in Fig. 1, the injection catheter 52 injects the liquid from an injection port 521 into the body cavity such as the subarachnoid space where the cerebrospinal fluid is present. In Fig. 1, as an example, the discharge catheter 51 and the injection catheter 52 are inserted from the first lumbar vertebra L1 from the top, and the discharge port 511 at the distal end position of the discharge catheter 51 is disposed near the eleventh thoracic vertebra T11 from the top. However, the insertion position and the distal end position of each of the discharge catheter 51 and the injection catheter 52 are not particularly limited as long as the arrangement ensures safety at the time of insertion.

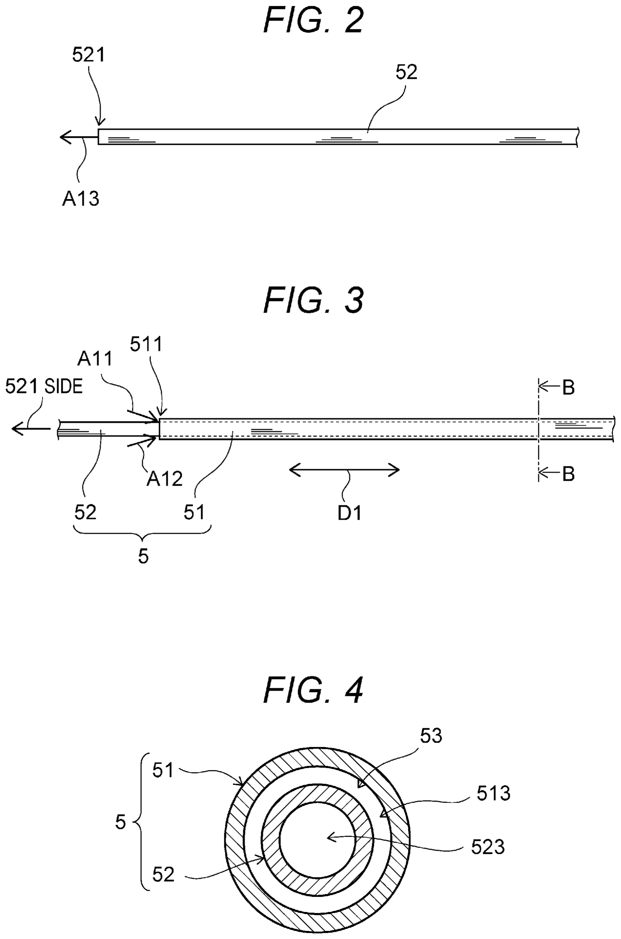

Fig. 2 is a plan view illustrating the vicinity of the injection port of the injection catheter of the present embodiment.

Fig. 3 is a plan view illustrating the vicinity of the discharge port of the discharge catheter of the present embodiment.

Fig. 4 is a cross-sectional view taken along the cross section B-B illustrated in Fig. 3.

As illustrated in Fig. 3, a distal end portion of the discharge catheter 51 is opened as the discharge port 511 and is disposed in the subarachnoid space in the vicinity of the lumbar vertebrae. For example, as indicated by arrows A11 and A12 illustrated in Fig. 3, the discharge catheter 51 is inserted into the subarachnoid space of the subject, and draws the cerebrospinal fluid present in the subarachnoid space in the vicinity of the lumbar vertebrae into a space 53 (see Fig. 4) between a lumen 513 of the discharge catheter 51 and an outer surface of the injection catheter 52 through the discharge port 511. Note that a force for drawing the cerebrospinal fluid is given by the pump 22 as described above with respect to Fig. 1. Then, as indicated by the arrow A1 illustrated in Fig. 1, the discharge catheter 51 discharges the cerebrospinal fluid out of the body cavity of the subject through the space 53.

An outer diameter of the injection catheter 52 is smaller than an inner diameter of the discharge catheter 51. The injection catheter 52 can be disposed in the lumen 513 of the discharge catheter 51. In addition, the injection catheter 52 is not coupled to the discharge catheter 51, and is movable in the lumen 513 of the discharge catheter 51 along a longitudinal direction D1 (see Fig. 3) of the discharge catheter 51. Since the distal end portion of the discharge catheter 51 is opened as the discharge port 511, a distal end portion of the injection catheter 52 can pass through the discharge port 511 of the discharge catheter 51 as illustrated in Fig. 3.

As a result, the distal end portion of the injection catheter 52 can be exposed from the discharge port 511 of the discharge catheter 51 in the longitudinal direction D1 of the discharge catheter 51. A distance in the longitudinal direction D1 between the distal end portion of the discharge catheter 51 and the distal end portion of the injection catheter 52 exposed from the discharge port 511 of the discharge catheter 51 can be adjusted to a predetermined distance. The "predetermined distance" in the specification of the present application is, for example, 0 cm to about 30 cm. This makes it possible to avoid a risk that occurs when a catheter is deeply inserted into a subarachnoid space of a patient.

As illustrated in Fig. 2, the distal end portion of the injection catheter 52 is opened as the injection port 521, passes through the discharge port 511 of the discharge catheter 51, and is disposed in the subarachnoid space. For example, as indicated by an arrow A13 illustrated in Fig. 2, the injection catheter 52 is inserted into the subarachnoid space of the patient and injects the liquid into cerebrospinal fluid present in the subarachnoid space through the lumen 523 (see Fig. 4) of the injection catheter 52. Note that a force for injecting the liquid into the cerebrospinal fluid is given by the pump 22 as described above with respect to Fig. 1.

Next, flows of a liquid generated in a body cavity in liquid circulation will be described with reference to the drawings.

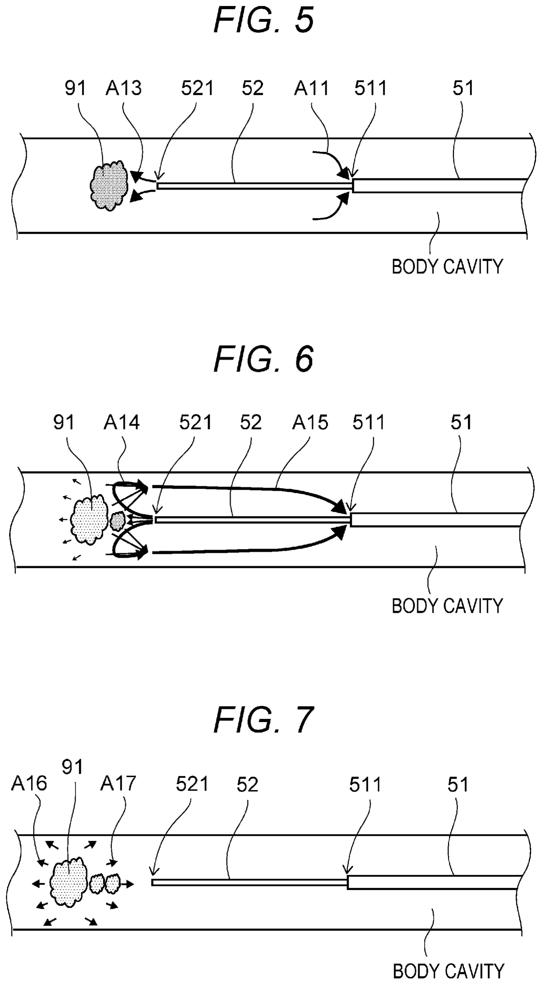

Fig. 5 is a schematic view illustrating a flow of the liquid generated in the body cavity immediately after the start of the liquid circulation.

Fig. 6 is a schematic view illustrating a flow of the liquid generated in the body cavity during the liquid circulation.

Fig. 7 is a schematic view illustrating a flow of the liquid generated in the body cavity after the stop of the liquid circulation.

As illustrated in Fig. 5, immediately after the start of the liquid circulation, a liquid 91 injected into the body cavity from the injection port 521 of the injection catheter 52 flows forward (that is, in a direction in which a brain is present) as indicated by an arrow A13 illustrated in Fig. 5, for example, until a flow of drawing when the cerebrospinal fluid is discharged from the discharge port 511 of the discharge catheter 51 reaches the injection port 521 as will be described below with reference to Fig. 6. A flow rate of the liquid 91 flowing forward is slightly larger than that in a case to be described below with reference to Fig. 6. In addition, in a short time immediately after the start of the liquid circulation, diffusion due to a concentration difference of oxygen or a drug (hereinafter, referred to as "oxygen or the like" for convenience of description) contained in the liquid hardly occurs.

Subsequently, as illustrated in Fig. 6, during the liquid circulation, that is, during continuous circulation of the liquid, the flow of drawing when the cerebrospinal fluid is discharged from the discharge port 511 of the discharge catheter 51 reaches the injection port 521 of the injection catheter 52 to exert an influence. Then, a flow from the injection port 521 of the injection catheter 52 toward the discharge port 511 of the discharge catheter 51 becomes steady. That is, a locally steady flow in which the liquid 91 injected into the body cavity from the injection port 521 of the injection catheter 52 flows toward the discharge port 511 of the discharge catheter 51 is generated, for example, as indicated by arrows A14 and A15 illustrated in Fig. 6. As a result, the liquid 91 injected into the body cavity from the injection port 521 of the injection catheter 52 hardly flows forward (that is, in the direction in which the brain is present).

**In** addition, due to an influence of the locally steady flow, diffusion of oxygen or the like contained in the liquid 91 to the brain side is decelerated as compared with the time immediately after the start of the liquid circulation. Due to the influence of the locally steady flow, the liquid 91 diffused to the lumbar side due to the concentration difference of oxygen or the like contained in the liquid 91 is discharged from the discharge port 511 of the discharge catheter 51 out of the body cavity.

As illustrated in Fig. 7, the locally steady flow generated during the liquid circulation disappears after the stop of the liquid circulation. Therefore, for example, as indicated by arrows A16 and A17 illustrated in Fig. 7, oxygen or the like contained in the liquid 91 is diffused to the brain side and the lumbar side.

As described with respect to Fig. 6, the locally steady flow in which the liquid 91 injected into the body cavity from the injection port 521 of the injection catheter 52 flows toward the discharge port 511 of the discharge catheter 51 is generated when the continuous circulation of the liquid is executed. As a result, a flow of the liquid 91 from the injection port 521 of the injection catheter 52 toward a treatment area of the brain decreases, and it becomes difficult to efficiently deliver the liquid 91 to the treatment area of the brain.

Therefore, the control unit 21 of the medical system 2 according to the present embodiment sets a first period in which the liquid 91 is injected into the body cavity and the liquid 91 is not discharged from the body cavity and a second period in which the liquid 91 is not injected into the body cavity and the liquid 91 is discharged from the body cavity, and alternately repeats the first period and the second period, so that the liquid 91 can be efficiently delivered to the brain side without generating the locally steady flow. In addition, by setting the injection amount of the liquid 91 in the first period to be substantially the same as the discharge amount of the liquid 91 in the second period, the control unit 21 can suppress the increase or decrease of the cerebrospinal fluid amount originally in the body cavity and suppress the pressure in the body cavity within the allowable range. Hereinafter, details of control of the medical system 2 according to the present embodiment will be described with reference to the drawings.

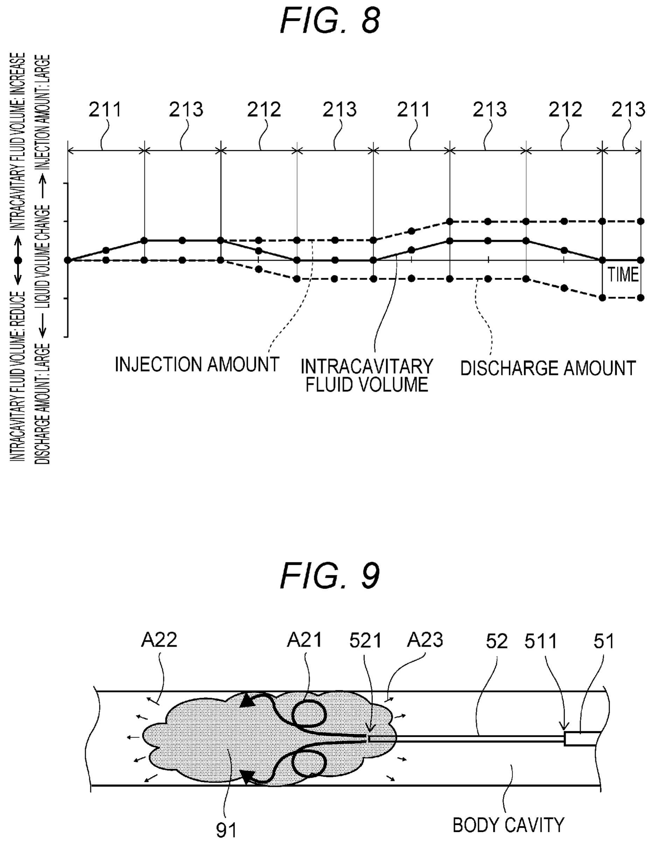

Fig. 8 is a graph showing a relationship among an injection amount, a discharge amount, and an intracavitary fluid volume by control of the present embodiment.

Fig. 9 is a schematic view illustrating a flow of the liquid generated in the body cavity by injection in control of the present embodiment.

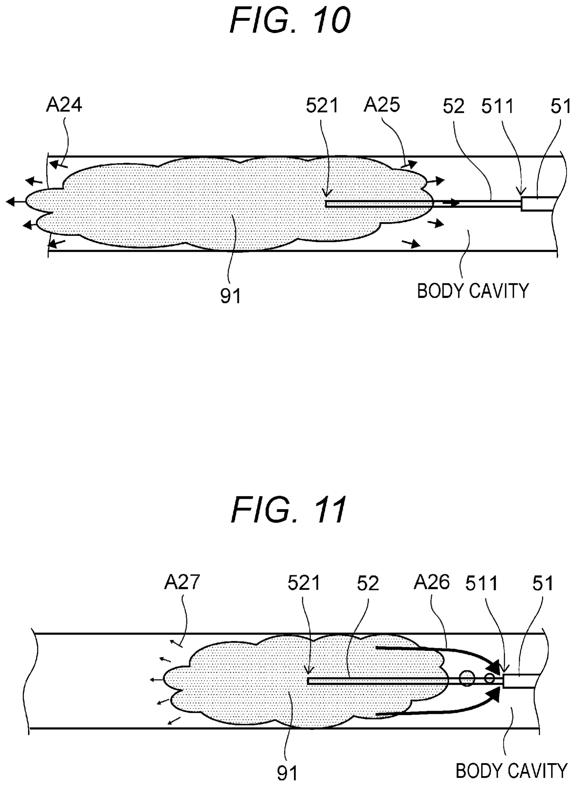

Fig. 10 is a schematic view illustrating a flow of the liquid generated in the body cavity during the stop of control of the present embodiment.

Fig. 11 is a schematic view illustrating a flow of the liquid generated in the body cavity by discharge in control of the present embodiment.

The horizontal axis of the graph shown in Fig. 8 represents time. The vertical axis of the graph shown in Fig. 8 represents the transition of the liquid amount. Specifically, the "injection amount" illustrated in Fig. 8 represents an integrated amount of the liquid 91 injected into the body cavity from the start of execution of the control. The "discharge amount" illustrated in Fig. 8 represents an integrated amount of the cerebrospinal fluid discharged out of the body cavity from the start of execution of the control. The "intracavitary fluid volume" illustrated in Fig. 8 represents a change amount of the cerebrospinal fluid present in the body cavity at the start of execution of the control.

As illustrated in Fig. 8, in the control of the present embodiment, the control unit 21 sets a first period 211 in which the liquid 91 is injected into the body cavity and the liquid 91 is not discharged from the body cavity, and a second period 212 in which the liquid 91 is not injected into the body cavity and the liquid 91 is discharged from the body cavity. That is, in the first period 211, only the injection of the liquid 91 into the body cavity is executed. On the other hand, in the second period 212, only the discharge of the liquid 91 from the body cavity is executed.

Then, as illustrated in Fig. 8, the control unit 21 alternately repeats the first period 211 and the second period 212. In addition, the control unit 21 sets the injection amount of the liquid 91 in the first period 211 to be substantially the same as the discharge amount of the liquid 91 in the second period 212. As a result, the injection of the liquid 91 and the discharge of the liquid 91 are alternately repeated in the same amount as long as the allowable fluctuation range of the intracranial pressure is not exceeded.

In the control of the medical system 2 according to the present embodiment, the injection and discharge of the liquid 91 are not simultaneously performed, and it is possible to suppress the occurrence of a locally steady flow in which the liquid 91 injected into the body cavity from the injection port 521 of the injection catheter 52 flows toward the discharge port 511 of the discharge catheter 51.

As illustrated in Fig. 9, in the first period 211, the liquid 91 injected into the body cavity from the injection port 521 of the injection catheter 52 flows to the brain side according to an injection direction and an injection flow rate. In addition, for example, as indicated by an arrow A21 illustrated in Fig. 9, the liquid 91 and the cerebrospinal fluid are stirred by turbulence generated by resistance between the liquid 91 injected into the body cavity from the injection port 521 of the injection catheter 52 and the cerebrospinal fluid in the body cavity. Furthermore, the diffusion due to the concentration difference of oxygen or the like contained in the liquid 91 progresses, for example, as indicated by arrows A22 and A23 in Fig. 9. As a result, the liquid can be efficiently delivered to a treatment area of a brain. In addition, since the injection amount of the liquid 91 in the first period 211 is substantially the same as the discharge amount of the liquid 91 in the second period 212, the amount of the liquid present in the body cavity is kept within a certain range. This can suppress fluctuations in intracranial pressure within the allowable range.

For example, as indicated by an arrow A26 illustrated in Fig. 11, in the second period 212, the same amount of the liquid 91 as the amount of the liquid 91 injected into the body cavity from the injection port 521 of the injection catheter 52 flows toward the discharge port 511 of the discharge catheter 51 and is drawn into the discharge port 511 of the discharge catheter 51. In addition, the liquid 91 injected into the body cavity from the injection port 521 of the injection catheter 52 is pulled back toward the discharge port 511 of the discharge catheter 51 as a whole, and the liquid 91 spread in the injection direction remains in the body cavity, for example, as indicated by an arrow A27 illustrated in Fig. 11.

As illustrated in Fig. 8, the control unit 21 further sets a third period 213 in which the liquid 91 is not injected into the body cavity and the liquid 91 is not discharged from the body cavity between the first period 211 and the second period 212 and between the second period 212 and the first period 211. That is, in the third period 213, the injection of the liquid 91 into the body cavity and the discharge of the liquid 91 from the body cavity are simultaneously stopped. The control unit 21 repeats the first period 211, the third period 213, the second period 212, and the third period 213 in this order. In the third period, the diffusion due to the concentration difference of oxygen or the like contained in the liquid 91 progresses toward the brain side and the lumbar side, for example, as indicated by arrows A24 and A25 in Fig. 10, according to the duration of the third period 213 (that is, stop time). The duration of the third period 213 is preferably until the flow generated by the immediately preceding injection or discharge disappears, and specifically, 1 second or more is preferable.

As described above, in the control of the medical system 2 according to the present embodiment, the injection and discharge of the liquid 91 are not simultaneously performed, and it is possible to suppress the occurrence of a locally steady flow in which the liquid 91 injected into the body cavity from the injection port 521 of the injection catheter 52 flows toward the discharge port 511 of the discharge catheter 51. Therefore, the liquid 91 injected into the body cavity flows toward the treatment area of the brain according to the injection direction and the injection flow rate. In addition, the liquid and the cerebrospinal fluid are stirred by the turbulence generated by the resistance between the liquid injected into the body cavity and the cerebrospinal fluid in the body cavity. Furthermore, diffusion due to a concentration difference of oxygen or the like contained in the liquid 91 proceeds. As a result, the liquid can be efficiently delivered to a treatment area of a brain. In addition, since the injection amount of the liquid 91 is substantially the same as the discharge amount of the liquid 91, the amount of the liquid existing in the body cavity can be maintained within a certain range. This can suppress fluctuations in intracranial pressure within the allowable range.

A cycle from the start of the first period 211 to the end of the second period 212 is substantially constant during the execution of the control. Therefore, at least one of stirring of the liquid 91 and the cerebrospinal fluid and diffusion due to a concentration difference of oxygen or the like contained in the liquid 91 stably progresses. As a result, a therapeutic substance (oxygen or the like) can be efficiently delivered to the treatment area of the brain. In addition, the amount of the liquid present in the body cavity can be stably kept in the certain range. This can suppress fluctuations in intracranial pressure within the allowable range.

Furthermore, as described above, the control unit 21 further sets the third period 213 in which the liquid 91 is not injected into the body cavity and the liquid 91 is not discharged from the body cavity between the first period 211 and the second period 212 and between the second period 212 and the first period 211. Therefore, the diffusion of oxygen and the like contained in the liquid 91 further proceeds according to the length of the third period. As a result, the liquid can be more efficiently delivered to a treatment area of a brain.

The discharge rate of the liquid 91 in the second period 212 may be slower than the injection rate of the liquid 91 in the first period 211. In this case, since the discharge rate in the second period 212 is slower than the injection rate in the first period 211, it is possible to suppress the liquid 91 immediately after being injected into the body cavity from the injection port 521 of the injection catheter 52 from being discharged from the discharge port 511 of the discharge catheter 51 to the outside of the body cavity. As a result, a therapeutic substance (oxygen or the like) can be efficiently delivered to the treatment area of the brain.

As illustrated in Fig. 8, the control unit 21 sets the second period 212 at the end or just before the end of the control, and executes control to approach the amount of cerebrospinal fluid existing in the body cavity at the start of execution of the control by discharging the liquid from the body cavity. According to this, the control unit 21 can set the amount of liquid present in the body cavity at the end of the control of the medical system 2 to be substantially the same as the amount of cerebrospinal fluid present in the body cavity at the start of the execution of the control of the medical system 2. Therefore, the intracranial pressure at the end of the control of the medical system 2 can be set to be substantially the same as the intracranial pressure at the start of the execution of the control of the medical system 2. As a result, it is possible to suppress the fluctuation in the intracranial pressure between the end of the control of the medical system 2 and the start of the execution of the control of the medical system 2.

Note that the control unit 21 may perform control so as to change the order of first period 211 and second period 212 to start from second period 212 (that is, liquid 91 is discharged). In this case, the first period 211 is set at the end or just before the end of the control, and the control is executed to approach the amount of cerebrospinal fluid existing in the body cavity at the start of execution of the control by injecting the liquid into the body cavity. Even in this case, an effect similar to that in the case where the control unit 21 sets the second period 212 at the end of the control or just before the end of the control can be obtained.

Next, a modification of control of the medical system according to the present embodiment will be described with reference to the drawings.

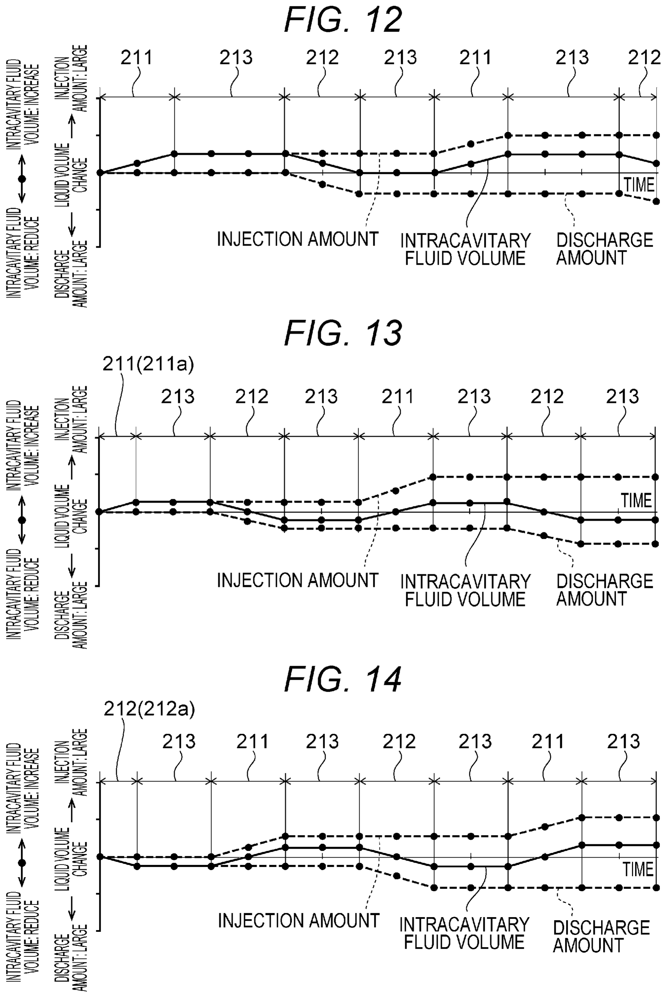

Fig. 12 is a graph showing a relationship among an injection amount, a discharge amount, and an intracavitary fluid volume of a first modification in control of the present embodiment.

The horizontal and vertical axes of the graph illustrated in Fig. 12 and "injection amount", "discharge amount", and "intracavitary fluid volume" illustrated in Fig. 12 are the same as those described above with respect to Fig. 8.

As illustrated in Fig. 12, in the first modification of the control of the present embodiment, the control unit 21 repeats the first period 211, the third period 213, the second period 212, and the third period 213 in this order. As illustrated in Fig. 12, the duration of the third period 213 set between the first period 211 and the second period 212 is longer than the duration of the first period 211. According to this, the time during which diffusion due to the concentration difference of oxygen or the like contained in the liquid 91 progresses is longer than the injection time of the liquid 91. As a result, the liquid can be more efficiently delivered to a treatment area of a brain.

As illustrated in Fig. 12, the duration (first time) of the third period 213 set between the first period 211 and the second period 212 is longer than the duration (second time) of the third period 213 set between the second period 212 and the first period 211. According to this, it is possible to more reliably suppress the start of the discharge by the discharge catheter 51 before the flow of the liquid 91 injected into the body cavity from the injection port 521 of the injection catheter 52 is stopped, and to reliably secure the time during which the diffusion due to the concentration difference of oxygen or the like contained in the liquid 91 progresses. As a result, the liquid can be more efficiently delivered to a treatment area of a brain. In addition, by shortening the duration (second time) of the third period 213 set between the second period 212 and the first period 211, the timing of continuously injecting the liquid 91 containing oxygen or the like as a therapeutic substance can be advanced, so that it is expected to enhance the therapeutic effect.

Fig. 13 is a graph showing a relationship among an injection amount, a discharge amount, and an intracavitary fluid volume of a second modification in control of the present embodiment.

The horizontal and vertical axes of the graph illustrated in Fig. 13 and "injection amount", "discharge amount", and "intracavitary fluid volume" illustrated in Fig. 13 are the same as those described above with respect to Fig. 8.

As shown in Fig. 13, in the second modification of the control of the present embodiment, the control unit 21 sets a 0th period 211a for injecting the liquid 91 in an amount smaller than the injection amount in the first period 211 as the initial period, and then repeats the third period 213, the second period 212, the third period 213, and the first period 211 in this order. As illustrated in Fig. 13, the injection amount of the liquid 91 in the initial 0th period 211a is smaller than the injection amount of the liquid 91 in the second and subsequent first periods 211. For example, the injection amount of the liquid 91 in the initial 0th period 211a is substantially half the injection amount of the liquid 91 in the second and subsequent first periods 211.

As illustrated in Fig. 13, the control unit 21 sets the injection time of the liquid 91 in the initial 0th period 211a to be shorter than the injection time of the liquid 91 in the second and subsequent first periods 211, thereby setting the injection amount of the liquid 91 in the initial 0th period 211a to be smaller than the injection amount of the liquid 91 in the second and subsequent first periods 211. Alternatively, the control unit 21 may set the injection rate of the liquid 91 in the initial 0th period 211a to be slower than the injection rate of the liquid 91 in the second and subsequent first periods 211, thereby setting the injection amount of the liquid 91 in the initial 0th period 211a to be smaller than the injection amount of the liquid 91 in the second and subsequent first periods 211. Furthermore, when the control is ended or is about to be ended, the same amount of liquid as that in the 0th period 211a is discharged and the control is ended, so that the final pressure in the body cavity can be made substantially the same as the pressure at the start of execution of the control and the control can be ended.

According to the present modification, since the cerebrospinal fluid volume in the body cavity fluctuates up and down across the original amount, the displacement of the fluctuation from the intracranial pressure at the start of execution of control can be made smaller than that of the above-described embodiment. Alternatively, after the second time, the amount of the liquid 91 containing the therapeutic substance injected at one time can be larger than that of the above-described embodiment. As a result, the fluctuation in the intracranial pressure can be suppressed within an allowable fluctuation range, and a higher therapeutic effect can be obtained. Also in the present modification, similarly to the modification shown in Fig. 12, the duration (first time) of the stop period (third period) between the injection and the discharge may be set longer than the duration (second time) of the stop period (third period) between the discharge and the injection.

Fig. 14 is a graph showing a relationship among an injection amount, a discharge amount, and an intracavitary fluid volume of a third modification in control of the present embodiment.

The horizontal and vertical axes of the graph illustrated in Fig. 14 and "injection amount", "discharge amount", and "intracavitary fluid volume" illustrated in Fig. 14 are the same as those described above with respect to Fig. 8.

As shown in Fig. 14, in the third modification of the control of the present embodiment, the control unit 21 sets the 0th period 212a for discharging the liquid 91 in an amount smaller than the discharge amount in the second period 212 as the initial period, and then repeats the third period 213, the first period 211, the third period 213, and the second period 212 in this order. As illustrated in Fig. 14, the discharge amount of the liquid 91 in the initial 0th period 212a is smaller than the discharge amount of the liquid 91 in the second and subsequent second periods 212. For example, the discharge amount of the liquid 91 in the initial 0th period 212a is substantially half the discharge amount of the liquid 91 in the second and subsequent second periods 212.

As illustrated in Fig. 14, the control unit 21 sets the discharge amount of the liquid 91 in the initial 0th period 212a to an amount smaller than the discharge amount of the liquid 91 in the second and subsequent second periods 212 by setting the discharge time of the liquid 91 in the initial 0th period 212a to a time shorter than the discharge time of the liquid 91 in the second and subsequent second periods 212. Alternatively, the control unit 21 may set the discharge amount of the liquid 91 in the initial 0th period 212a to an amount smaller than the discharge amount of the liquid 91 in the second and subsequent second periods 212 by setting the discharge rate of the liquid 91 in the initial 0th period 212a to a rate slower than the discharge rate of the liquid 91 in the second and subsequent second periods 212. In addition, when the control is ended or is about to be ended, by injecting the same amount of liquid as that in the 0th period 212a and ending the control, it is possible to make the final pressure in the body cavity substantially the same as the pressure at the start of execution of the control and end the control.

According to the present modification, since the cerebrospinal fluid volume in the body cavity fluctuates up and down across the original amount, the displacement of the fluctuation from the intracranial pressure at the start of execution of control can be made smaller than that of the above-described embodiment. Alternatively, the amount of the liquid 91 containing the therapeutic substance injected at a time can be greater than that of the above-described embodiments. As a result, the fluctuation in the intracranial pressure can be suppressed within an allowable fluctuation range, and a higher therapeutic effect can be obtained. Also in the present modification, similarly to the modification shown in Fig. 12, the duration (first time) of the stop period (third period) between the injection and the discharge may be set longer than the duration (second time) of the stop period (third period) between the discharge and the injection.

Next, an example of an experiment performed by the present inventor will be described with reference to the drawings.

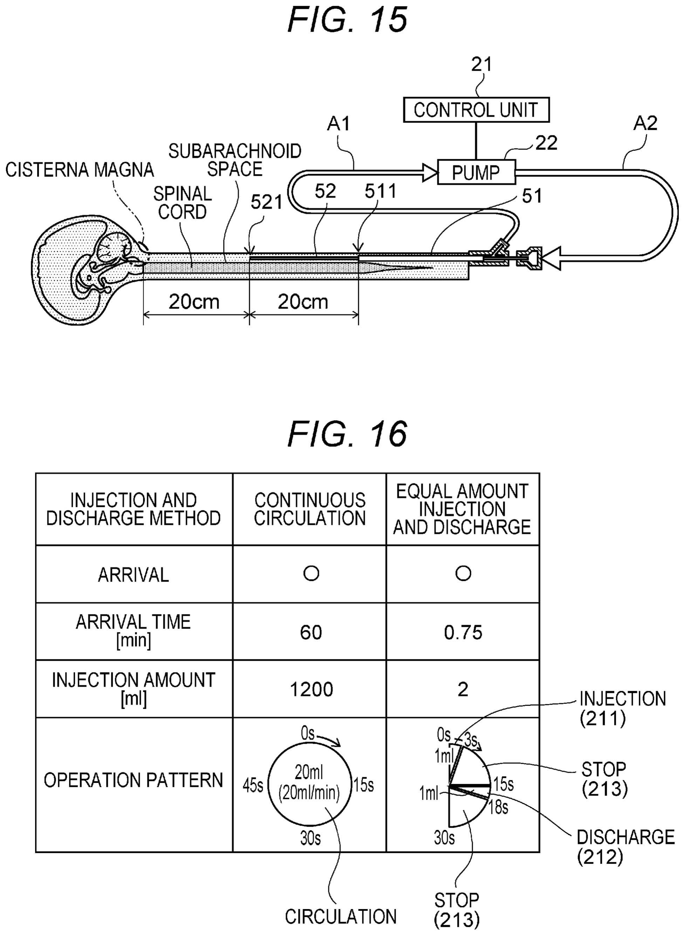

Fig. 15 is a schematic diagram illustrating an outline of the experiment performed by the present inventor.

Fig. 16 is a table showing an example of results of the experiment performed by the present inventor.

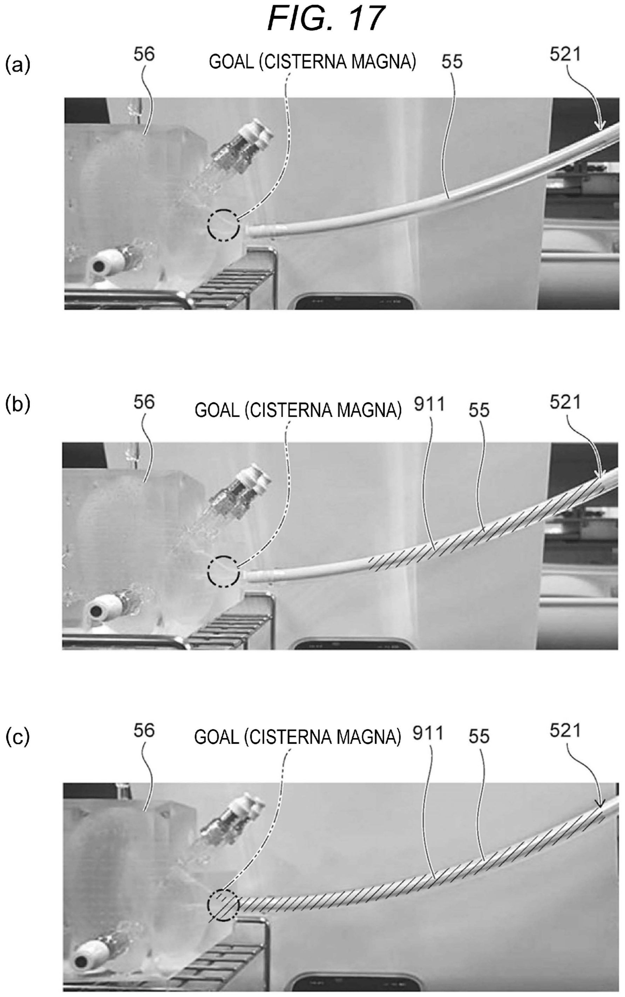

Fig. 17 is a set of photographs showing states of the experiment performed by the present inventor.

Fig. 17(a) is a photograph showing a state at the start of the experiment. Fig. 17(b) is a photograph showing a state in the middle of the experiment, that is, a state in which colored water 911 arrives at an intermediate position of a tube 55. Fig. 17(c) is a photograph showing a state in which the colored water 911 arrives at a goal position. Note that the colored water 911 is hatched in the photographs of Figs. 17(b) and 17(c) for convenience of description.

The present inventor injected the colored water 911 into the tube 55 from the injection port 521 of the injection catheter 52 using a model imitating a spinal cord portion of a subarachnoid space as illustrated in Figs. 17(a) to 17(c), and compared times required from the injection of the colored water 911 into the tube 55 to arrival at a position set as a goal. In the model illustrated in Figs. 17(a) to 17(c), a lumen of the tube 55 is a portion imitating the subarachnoid space. A lumen of a tank 56 is a portion imitating an intracranial space. The goal position is a position near a connection portion between the tube 55 and the tank 56, and is a position immediately after the colored water 911 having passed through the tube 55 flows into the tank 56. The goal position is a portion imitating cisterna magna.

As illustrated in Fig. 15, the distance in the longitudinal direction D1 (see Fig. 3) between the distal end portion (that is, discharge port 511) of the discharge catheter 51 and the distal end portion (that is, injection port 521) of the injection catheter 52 exposed from the discharge port 511 of the discharge catheter 51 is 20 cm. In addition, a distance in the longitudinal direction D1 between the injection port 521 of the injection catheter 52 and the goal position (that is, portion imitating the cisterna magna) is 20 cm.

An injection and discharge method in the experiment will be described with reference to Fig. 16.

"Continuous circulation" in the table shown in Fig. 16 is a method of continuously injecting and discharging the colored water 911, and is a method as a comparative example. That is, as in the table shown in Fig. 16, in the "continuous circulation", the control unit 21 sets a period for continuously injecting and discharging the colored water 911 at a flow rate of 20 mL/min.

In the table shown in Fig. 16, "equal amount injection and discharge" is an injection and discharge method when the control described above with reference to Figs. 8 to 11 is executed. That is, as in the table shown in Fig. 16, in the "equal amount injection and discharge", the control unit 21 sets the first period 211 in which the colored water 911 is injected into the tube 55 and the colored water 911 is not discharged from the tube 55, and the second period 212 in which the colored water 911 is not injected into the tube 55 and the colored water 911 is discharged from the tube 55. The duration of the first period 211 is 3 seconds. In the first period 211, the colored water 911 at a flow rate of 20 mL/min is injected into the tube 55 for 3 seconds, so that an injection amount of the colored water 911 is 1 mL. The duration of the second period 212 is 3 seconds. In the second period 212, the colored water 911 at a flow rate of 20 mL/min is discharged from the tube 55 for 3 seconds, so that a discharge amount of the colored water 911 is 1 mL. Then, the control unit 21 alternately repeats the first period 211 and the second period 212. Furthermore, in the "equal amount injection and discharge", the control unit 21 sets the third period 213 in which the colored water 911 is not injected into the tube 55 and the colored water 911 is not discharged from the tube 55 between the first period 211 and the second period 212 and between the second period 212 and the first period 211. The duration of the third period 213 is 12 seconds. In this manner, in the "equal amount injection and discharge", the control unit 21 repeats the first period 211, the third period 213, the second period 212, and the third period 213 in this order.

An example of results of the experiment is shown in the table of Fig. 16. That is, the colored water 911 could arrive at the goal position in both the "continuous circulation" and the "equal amount injection and discharge". A time required for the colored water 911 to arrive at the goal position after being injected into the tube 55 is 60 minutes in the "continuous circulation" and 0.75 minutes in the "equal amount injection and discharge". The amount of the colored water 911 injected until the colored water 911 arrives at the goal position after being injected into the tube 55 is 1200 mL in the "continuous circulation" and 2 mL in the "equal amount injection and discharge". From the results of the experiment, it can be seen that the "equal amount injection and discharge" can efficiently deliver the colored water 911 to the goal position as compared with the "continuous circulation".

Next, a liquid circulation system according to the present embodiment will be described with reference to the drawings.

Note that, in a case where components of a liquid circulation system 3 are similar to the components of the medical system 2 described above with reference to Figs. 1 to 17, redundant description will be appropriately omitted, and differences will be mainly described hereinafter.

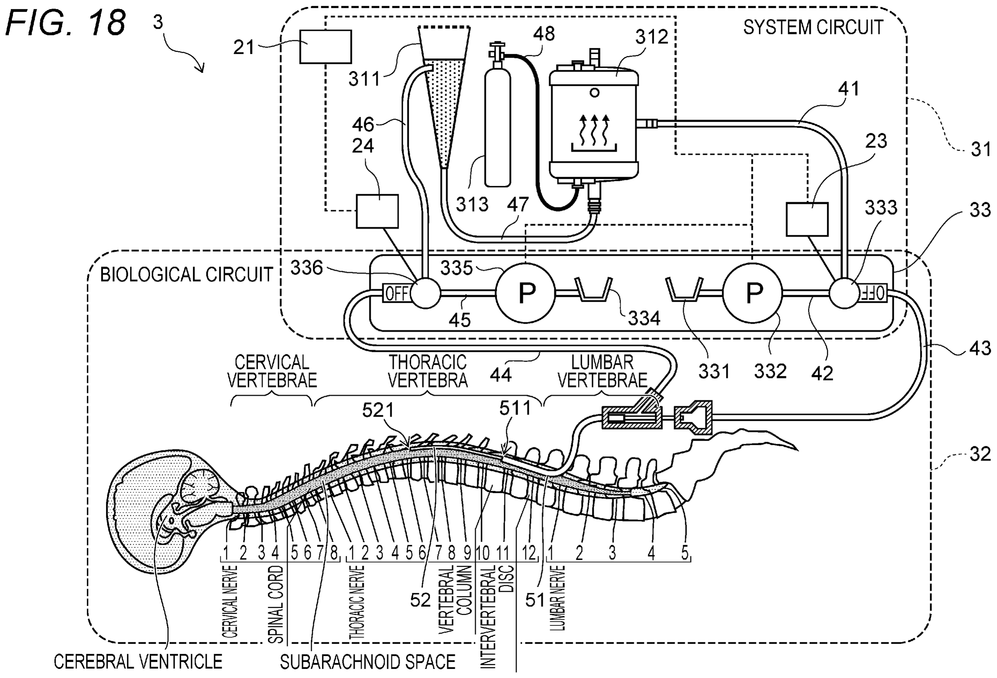

Fig. 18 is a schematic diagram illustrating an outline of the liquid circulation system according to the present embodiment.

The liquid circulation system 3 according to the present embodiment injects a liquid into a body cavity and discharges the liquid out of the body cavity to circulate the liquid. Examples of the inside of the body cavity include the inside of a body cavity in which cerebrospinal fluid of a subject is present. In the description of the liquid circulation system 3 according to the present embodiment, a case where the liquid to be injected into the body cavity is a high oxygen solution will be described as an example.

As illustrated in Fig. 18, the liquid circulation system 3 includes a system circuit 31, a biological circuit 32, and a pump unit 33.

The system circuit 31 is a portion that generates the high oxygen solution, adjusts a temperature, and adjusts a total volume of the liquid in a circulation circuit, and includes the control unit 21, a reservoir 311, an oxygenation mechanism 312, and an oxygen supply source 313. The oxygenation mechanism 312 of the present embodiment is an example of a "liquid treatment device" and an "oxygenator" of the present invention.

The system circuit 31 further includes a first drive unit 23 and a second drive unit 24. Each of the first drive unit 23 and the second drive unit 24 is connected to the control unit 21, and operates on the basis of a control signal transmitted from control unit 21. Examples of the first drive unit 23 and the second drive unit 24 include actuators such as motors. Note that the first drive unit 23 and the second drive unit 24 may be provided in the pump unit 33.

The biological circuit 32 is a portion that injects the liquid into the body cavity and discharges the liquid out of the body cavity, and includes the discharge catheter 51 and the injection catheter 52. The discharge catheter 51 and the injection catheter 52 are connected to the system circuit 31 via the pump unit 33. The discharge catheter 51 and the injection catheter 52 are the same as those described with respect to Figs. 1 to 17.

The pump unit 33 functions as a liquid delivery unit shared by the system circuit 31 and the biological circuit 32. The pump unit 33 includes a first liquid storage unit 331, a first pump 332, a first flow path switching unit 333, a second liquid storage unit 334, a second pump 335, and a second flow path switching unit 336. The first pump 332 of the present embodiment is an example of a "first liquid delivery unit" of the present invention. The second pump 335 of the present embodiment is an example of a "second liquid delivery unit" of the present invention. As described above, the pump unit 33 may include the first drive unit 23 and the second drive unit 24.

As illustrated in Fig. 18, the oxygenation mechanism 312 is connected to the first flow path switching unit 333 via a first tube 41. In addition, the oxygenation mechanism 312 is connected to the reservoir 311 via a seventh tube 47. Furthermore, the oxygenation mechanism 312 is connected to the oxygen supply source 313 via an eighth tube 48. The oxygenation mechanism 312 mixes oxygen supplied from the oxygen supply source 313 through the eighth tube with cerebrospinal fluid supplied from the reservoir 311 through the seventh tube 47 or artificial cerebrospinal fluid such as lactated Ringer's solution or a mixed solution of oxygen, cerebrospinal fluid, and an artificial cerebrospinal fluid such as lactated Ringer's solution to generate oxygenated cerebrospinal fluid. In addition, the oxygenation mechanism 312 also includes a heat exchanger that adjusts a temperature of the oxygenated cerebrospinal fluid. Then, the oxygenation mechanism 312 supplies the oxygenated cerebrospinal fluid as the high oxygen solution to the first flow path switching unit 333 through the first tube 41. As the oxygenation mechanism 312 in the present embodiment, a hollow fiber oxygenator for adding oxygen to blood can be used.

The first flow path switching unit 333 is provided at a connection portion among the first tube 41, a second tube 42, and a third tube 43, and can switch between a flow path in which the first tube 41 and the second tube 42 are connected and a flow path in which the second tube 42 and the third tube 43 are connected. Specifically, the first drive unit 23 controls an operation of the first flow path switching unit 333 on the basis of a control signal transmitted from the control unit 21 to switch between the flow path in which the first tube 41 and the second tube 42 are connected and the flow path in which the second tube 42 and the third tube 43 are connected. The first tube 41, the second tube 42, and the third tube 43 are an example of a "liquid delivery line" of the present invention, and are connected to the first liquid storage unit 331 and the injection catheter 52.

The first flow path switching unit 333 is connected to the first liquid storage unit 331 via the second tube 42. The first pump 332 is provided in the second tube 42. Examples of the first pump 332 include a roller pump and a syringe pump. The first pump 332 operates on the basis of a control signal transmitted from the control unit 21, and moves liquid toward the first liquid storage unit 331 or moves liquid toward the first flow path switching unit 333.

When the first pump 332 is driven in the first direction in a state where the first flow path switching unit 333 connects the first tube 41 and the second tube 42, the first pump 332 supplies the liquid (that is, high oxygen solution) generated in the oxygenation mechanism 312 to the first liquid storage unit 331 through the first tube 41 and the second tube 42. As a result, the first liquid storage unit 331 stores the liquid supplied from the oxygenation mechanism 312 through the first tube 41 and the second tube 42. When the first pump 332 is driven in the second direction in a state where the first flow path switching unit 333 connects the first tube 41 and the second tube 42, the first pump 332 supplies the liquid stored in the first liquid storage unit 331 to the oxygenation mechanism 312 through the first tube 41 and the second tube 42.

On the other hand, when the first pump 332 is driven in the second direction in a state where the first flow path switching unit 333 connects the second tube 42 and the third tube 43, the first pump 332 supplies the liquid stored in the first liquid storage unit 331 to the injection catheter 52 through the second tube 42 and the third tube 43.

As illustrated in Fig. 18, the reservoir 311 is connected to the second flow path switching unit 336 via a sixth tube 46. In addition, the reservoir 311 is connected to the oxygenation mechanism 312 via the seventh tube 47. The reservoir 311 temporarily stores the cerebrospinal fluid supplied through the sixth tube 46. Then, the reservoir 311 supplies the stored cerebrospinal fluid to the oxygenation mechanism 312 through the seventh tube 47. The reservoir 311 has a structure in which the inside and the outside communicate with each other, and can extract gas contained in the stored cerebrospinal fluid to the outside. That is, the reservoir 311 has a function as an air trap.

The second flow path switching unit 336 is provided at a connection portion among a fourth tube 44, a fifth tube 45, and the sixth tube 46, and can switch between a flow path in which the fourth tube 44 and the fifth tube 45 are connected and a flow path in which the fifth tube 45 and the sixth tube 46 are connected. Specifically, the second drive unit 24 controls an operation of the second flow path switching unit 336 on the basis of a control signal transmitted from the control unit 21 to switch between the flow path in which the fourth tube 44 and the fifth tube 45 are connected and the flow path in which the fifth tube 45 and the sixth tube 46 are connected. The fourth tube 44, the fifth tube 45, and the sixth tube 46 are an example of a "liquid discharge line" of the present invention, and are connected to the second liquid storage unit 334 and the discharge catheter 51.

The second flow path switching unit 336 is connected to the second liquid storage unit 334 via the fifth tube 45. The second pump 335 is provided in the fifth tube 45. Examples of the second pump 335 include a roller pump and a syringe pump. The second pump 335 operates on the basis of a control signal transmitted from the control unit 21, and moves liquid toward the second liquid storage unit 334 or moves liquid toward the second flow path switching unit 336.

When the second pump 335 is driven in the first direction in a state where the second flow path switching unit 336 connects the fourth tube 44 and the fifth tube 45, the second pump 335 supplies the liquid (that is, cerebrospinal fluid) discharged from the body cavity to the outside of the body cavity to the second liquid storage unit 334 through the fourth tube 44 and the fifth tube 45. As a result, the second liquid storage unit 334 stores the liquid (that is, cerebrospinal fluid) discharged from the body cavity to the outside of the body cavity through the fourth tube 44 and the fifth tube 45.

On the other hand, when the second pump 335 is driven in the first direction in a state where the second flow path switching unit 336 connects the fifth tube 45 and the sixth tube 46, the second pump 335 supplies the cerebrospinal fluid stored in the reservoir 311 to the second liquid storage unit 334 via the fifth tube 45 and the sixth tube 46. As a result, the second liquid storage unit 334 stores the liquid (that is, cerebrospinal fluid) supplied from the reservoir 311 through the fifth tube 45 and the sixth tube 46. When the second pump 335 is driven in the second direction in a state where the second flow path switching unit 336 connects the fifth tube 45 and the sixth tube 46, the second pump 335 supplies the liquid (that is, cerebrospinal fluid) stored in the second liquid storage unit 334 to the reservoir 311 through the fifth tube 45 and the sixth tube 46.

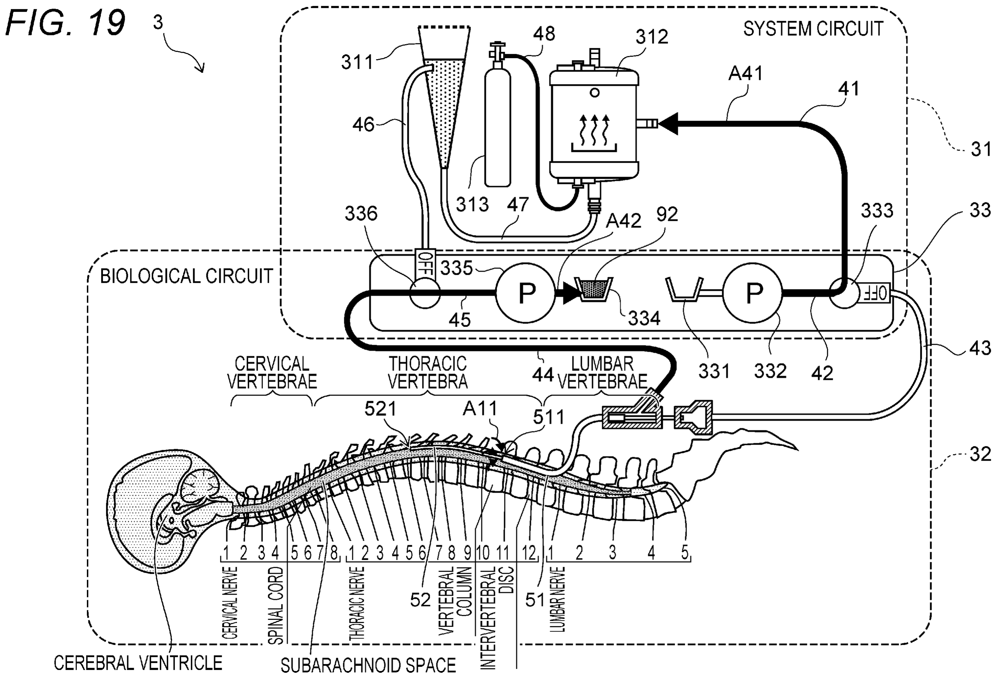

Figs. 19 to 22 are schematic diagrams for describing the operation of the liquid circulation system according to the present embodiment.

The operation of the liquid circulation system 3 described with reference to Figs. 19 to 22 is the operation based on the control described above with reference to Figs. 8 to 11.

Note that the control unit 21, the first drive unit 23, and the second drive unit 24 are omitted in Figs. 19 to 22 for convenience of description. In addition, the following operation will be described on the assumption that the tubes, the reservoir, and the oxygenation mechanism in the circuit start from a primed state filled with artificial cerebrospinal fluid such as lactated Ringer's solution in advance.

First, as illustrated in Fig. 19, as a first step, the control unit 21 transmits a control signal to the first drive unit 23 to control the operation of the first flow path switching unit 333 and connect the first tube 41 and the second tube 42. In addition, the control unit 21 transmits a control signal to the second drive unit 24 to control the operation of the second flow path switching unit 336 and connect the fourth tube 44 and the fifth tube 45. In this state, the control unit 21 transmits a control signal to the first pump 332 to drive the first pump 332 in the second direction, and transmits a control signal to the second pump 335 to drive the second pump 335 in the first direction.

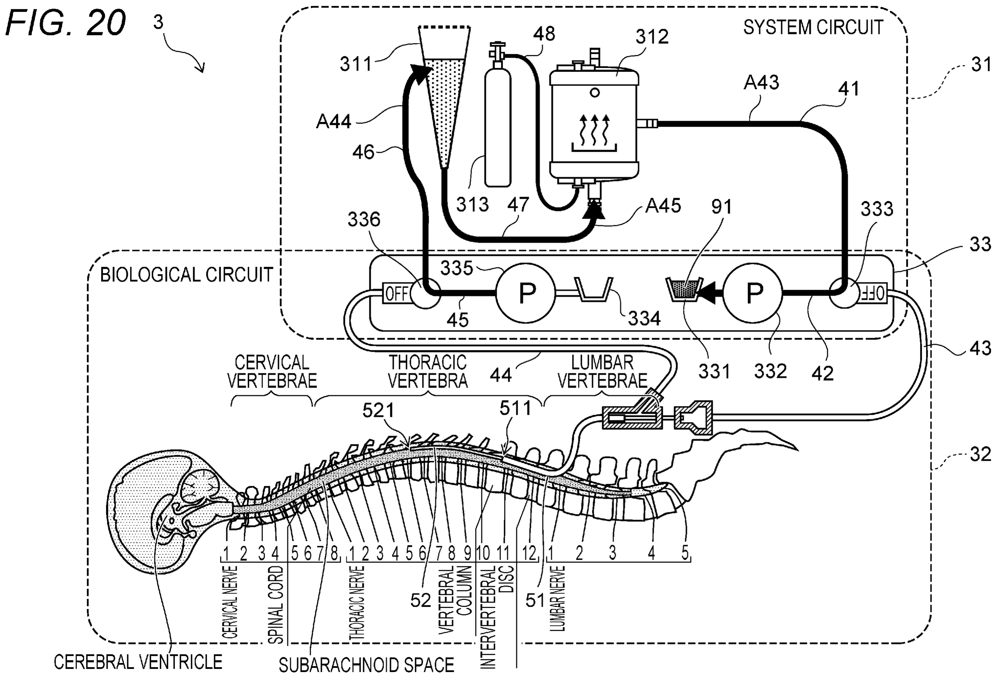

Then, as indicated by an arrow A41 illustrated in Fig. 19, the liquid 91 (see Fig. 20) stored in the first liquid storage unit 331 is returned to the oxygenation mechanism 312 through the first tube 41 and the second tube 42. On the other hand, for example, as indicated by an arrow A11 illustrated in Fig. 19, the cerebrospinal fluid 92 in the body cavity is drawn into the discharge port 511 of the discharge catheter 51. Then, as indicated by an arrow A42 illustrated in Fig. 19, the cerebrospinal fluid 92 is supplied to and stored in the second liquid storage unit 334 through the fourth tube 44 and the fifth tube 45.

Subsequently, as illustrated in Fig. 20, as a second step, the control unit 21 transmits a control signal to the first drive unit 23 to control the operation of the first flow path switching unit 333 and connect the first tube 41 and the second tube 42. In addition, the control unit 21 transmits a control signal to the second drive unit 24 to control the operation of the second flow path switching unit 336 and connect the fifth tube 45 and the sixth tube 46. In this state, the control unit 21 transmits a control signal to first pump 332 to drive first pump 332 in the first direction, and transmits a control signal to second pump 335 to drive second pump 335 in the second direction.

Then, as indicated by an arrow A43 illustrated in Fig. 20, the liquid 91 (that is, high oxygen solution) generated in the oxygenation mechanism 312 is supplied to and stored in the first liquid storage unit 331 through the first tube 41 and the second tube 42. In addition, as indicated by an arrow A44 illustrated in Fig. 20, the cerebrospinal fluid 92 stored in the second liquid storage unit 334 is supplied to and stored in the reservoir 311 through the fifth tube 45 and the sixth tube 46. Furthermore, as indicated by an arrow A45 illustrated in Fig. 20, the cerebrospinal fluid 92 stored in the reservoir 311 is supplied to the oxygenation mechanism 312 through the seventh tube 47.

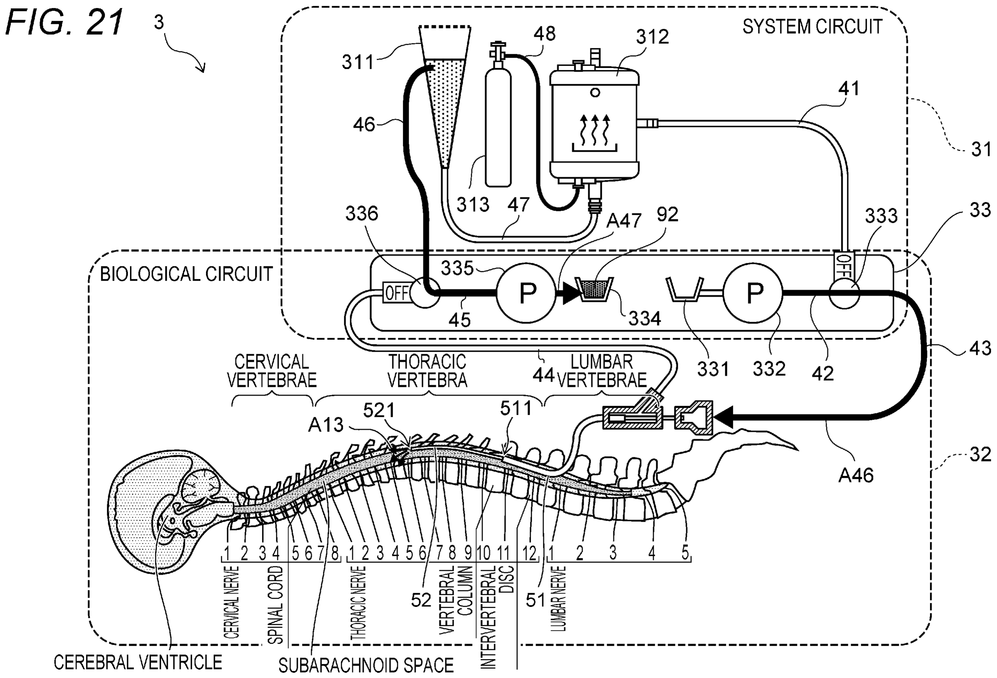

Subsequently, as illustrated in Fig. 21, as a third step, the control unit 21 transmits a control signal to the first drive unit 23 to control the operation of the first flow path switching unit 333 and connect the second tube 42 and the third tube 43. In addition, the control unit 21 transmits a control signal to the second drive unit 24 to control the operation of the second flow path switching unit 336 and connect the fifth tube 45 and the sixth tube 46. In this state, the control unit 21 transmits a control signal to the first pump 332 to drive the first pump 332 in the second direction, and transmits a control signal to the second pump 335 to drive the second pump 335 in the first direction.

Then, as indicated by an arrow A46 illustrated in Fig. 21, the liquid 91 (that is, high oxygen solution) stored in the first liquid storage unit 331 is supplied to the injection catheter 52 through the second tube 42 and the third tube 43. Then, for example, as indicated by an arrow A13 illustrated in Fig. 21, the liquid 91 is injected into the body cavity from the injection port 521 of the injection catheter 52. On the other hand, as indicated by an arrow A47 illustrated in Fig. 21, the cerebrospinal fluid 92 stored in the reservoir 311 is returned to the second liquid storage unit 334 through the fifth tube 45 and the sixth tube 46.

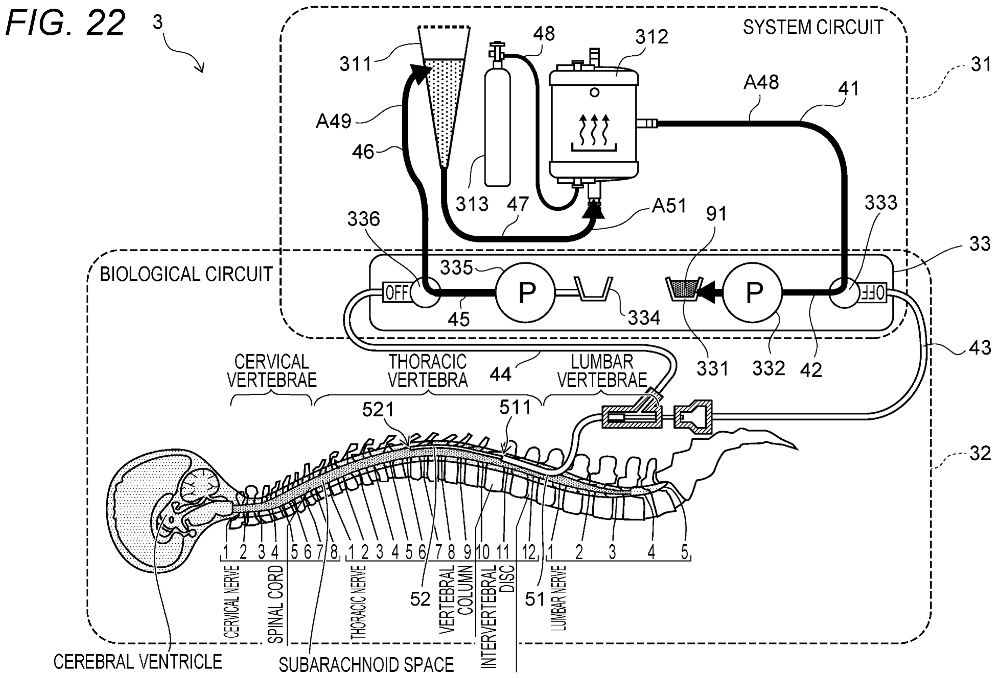

Subsequently, as illustrated in Fig. 22, as a fourth step, the control unit 21 transmits a control signal to the first drive unit 23 to control the operation of the first flow path switching unit 333 and connect the first tube 41 and the second tube 42. In addition, the control unit 21 transmits a control signal to the second drive unit 24 to control the operation of the second flow path switching unit 336 and connect the fifth tube 45 and the sixth tube 46. In this state, the control unit 21 transmits a control signal to first pump 332 to drive first pump 332 in the first direction, and transmits a control signal to second pump 335 to drive second pump 335 in the second direction.

Then, as indicated by an arrow A48 illustrated in Fig. 22, the liquid 91 (that is, high oxygen solution) generated in the oxygenation mechanism 312 is supplied to and stored in the first liquid storage unit 331 through the first tube 41 and the second tube 42. In addition, as indicated by an arrow A49 illustrated in Fig. 22, the cerebrospinal fluid 92 stored in the second liquid storage unit 334 is supplied to and stored in the reservoir 311 through the fifth tube 45 and the sixth tube 46. Furthermore, as indicated by an arrow A51 illustrated in Fig. 22, the cerebrospinal fluid 92 stored in the reservoir 311 is supplied to the oxygenation mechanism 312 through the seventh tube 47.

According to the liquid circulation system 3 of the present embodiment, the circuit for circulating liquid is divided into the circuit including the injection catheter 52 and the discharge catheter 51 (that is, biological circuit 32) and the circuit including the liquid delivery line (that is, first tube 41, second tube 42, and third tube 43) connected to the injection catheter 52 and the liquid discharge line (that is, fourth tube 44, fifth tube 45, and sixth tube 46) connected to the discharge catheter 51 (that is, system circuit 31). In addition, the first pump 332 that is provided on the liquid delivery line and moves the liquid and the second pump 335 that is provided on the liquid discharge line and moves the liquid function as a shared pump in the biological circuit 32 and the system circuit 31. Since the liquid circulation system 3 has such a configuration, the system circuit 31 can cope with an increase in the total volume of the liquid caused by the cavitation phenomenon and a decrease in the total volume of the liquid caused by evaporation while maintaining a situation on a living body side, and can suppress the increase and decrease in the amount of the liquid in the biological circuit. This can suppress fluctuations in intracranial pressure.

In addition, the control unit 21 repeatedly drives the first pump 332, the second pump 335, the first flow path switching unit 333, and the second flow path switching unit 336, and switches the direction of the flow of the liquid so as to perform the operations as illustrated in Figs. 19 to 22. As a result, the injection of the liquid 91 and the discharge of the cerebrospinal fluid 92 are alternately repeated in the same amount as long as an allowable fluctuation range of the intracranial pressure is not exceeded. Since the control unit 21 does not perform the control of the first flow path switching unit 333 to connect the second tube 42 and the third tube 43 and the second flow path switching unit 336 to connect the fourth tube 44 and the fifth tube 45 at the same time, the injection of the liquid 91 and the discharge of the cerebrospinal fluid 92 are not performed at the same time, and it is possible to suppress the generation of the locally steady flow in which the liquid 91 injected into the body cavity from the injection port 521 of the injection catheter 52 flows toward the discharge port 511 of the discharge catheter 51. Therefore, the liquid 91 injected into the body cavity flows toward the treatment area of the brain according to the injection direction and the injection flow rate. In addition, the liquid 91 and the cerebrospinal fluid 92 are stirred by the turbulence generated by the resistance between the liquid 91 injected into the body cavity and the cerebrospinal fluid 92 in the body cavity. Furthermore, diffusion due to a concentration difference of oxygen or the like contained in the liquid 91 proceeds. As a result, the liquid can be efficiently delivered to a treatment area of a brain.

In addition, the control unit 21 controls the sum of the injection amount of the liquid ejected toward the biological circuit 32 while the first pump 332 is continuously or intermittently activated in the third step and the discharge amount of the liquid ejected from the biological circuit 32 while the second pump 335 is continuously or intermittently activated in the first step from the time of initial activation within a certain range. According to this, it is possible to suppress the fluctuation in the amount of cerebrospinal fluid from the time of initial activation of the first pump 332 and the second pump 335 within a certain range. Therefore, it is possible to more reliably suppress the intracranial pressure from exceeding the allowable fluctuation range. This makes it possible to more reliably suppress the fluctuations in the intracranial pressure within the allowable fluctuation range.

Furthermore, when the injection destination into the body is the subarachnoid space and the liquid to be injected into the subarachnoid space is a high oxygen solution, the high oxygen solution can be efficiently delivered to the treatment area of the brain while suppressing the fluctuation in the intracranial pressure.

Next, a liquid circulation system according to the modification of the present embodiment will be described with reference to the drawings.

Note that, in a case where the components of the liquid circulation system 3A according to the first modification and the liquid circulation system 3B according to the second modification are similar to the components of the liquid circulation system 3 described above with reference to Figs. 18 to 22, the overlapping description will be appropriately omitted, and the difference will be mainly described below.

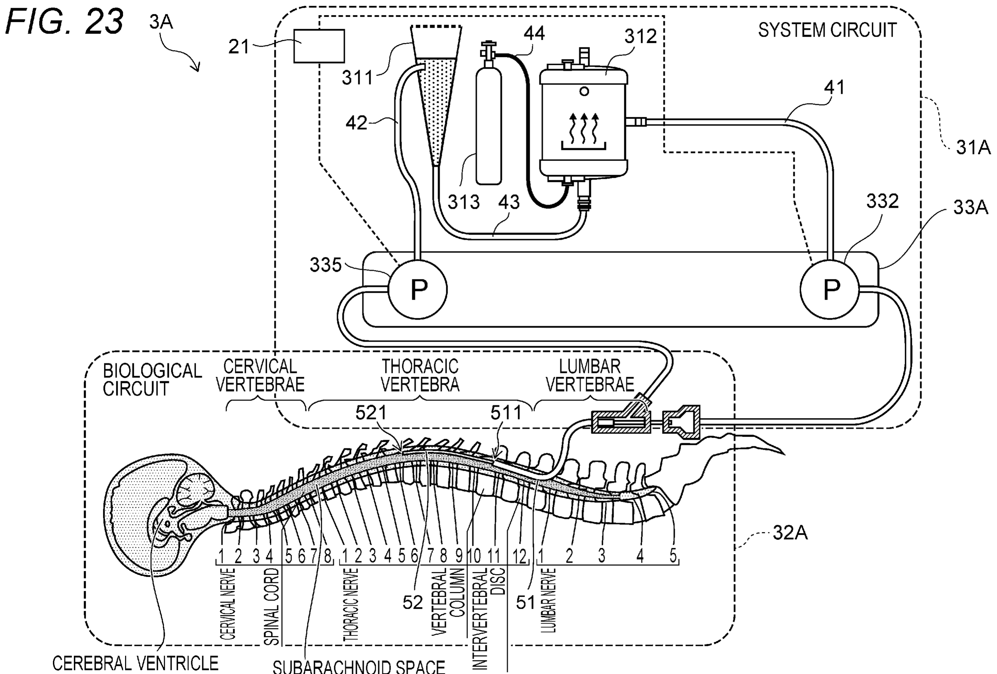

Fig. 23 is a schematic diagram illustrating an outline of a liquid circulation system according to the first modification of the present embodiment.

The liquid circulation system 3A according to the present modification injects a liquid into a body cavity and discharges the liquid out of the body cavity to circulate the liquid. Examples of the inside of the body cavity include the inside of a body cavity in which cerebrospinal fluid of a subject is present. In the description of the liquid circulation system 3A according to the present modification, a case where the liquid to be injected into the body cavity is a high oxygen solution will be described as an example.

As illustrated in Fig. 23, the liquid circulation system 3A includes a system circuit 31A, a biological circuit 32A, and a pump unit 33A.

The system circuit 31A is a portion that generates the high oxygen solution, adjusts a temperature, and adjusts a total volume of the liquid in a circulation circuit, and includes the control unit 21, a reservoir 311, an oxygenation mechanism 312, and an oxygen supply source 313. The oxygenation mechanism 312 of the present modification is an example of a "liquid treatment device" and an "oxygenator" of the present invention.

The biological circuit 32A is a portion that injects the liquid into the body cavity and discharges the liquid out of the body cavity, and includes the discharge catheter 51 and the injection catheter 52. The discharge catheter 51 and the injection catheter 52 are connected to the pump unit 33A and the system circuit 31A including the reservoir 311 and the oxygenation mechanism 312. The discharge catheter 51 and the injection catheter 52 are the same as those described with respect to Figs. 1 to 17.

The pump unit 33A functions as a liquid delivery unit shared by the system circuit 31A and the biological circuit 32A. The pump unit 33A includes the first pump 332 and the second pump 335. As the first pump 332 and the second pump 335, a roller pump that feeds the liquid in the tube by squeezing the liquid feeding tube with a roller or a peristaltic pump that feeds the liquid in the tube by squeezing the liquid feeding tube with a plurality of fingers can be used.

As illustrated in Fig. 23, the oxygenation mechanism 312 is connected to the injection catheter 52 via the first tube 41. In addition, the oxygenation mechanism 312 is connected to the reservoir 311 via the third tube 43. Furthermore, the oxygenation mechanism 312 is connected to the oxygen supply source 313 via the fourth tube 44. The oxygenation mechanism 312 mixes oxygen supplied from the oxygen supply source 313 through the fourth tube 44 with cerebrospinal fluid supplied from the reservoir 311 through the third tube 43 or lactated Ringer's solution or a mixed solution of oxygen, cerebrospinal fluid, and an artificial cerebrospinal fluid such as lactated Ringer's solution to generate oxygenated cerebrospinal fluid. In addition, the oxygenation mechanism 312 also includes a heat exchanger that adjusts a temperature of the oxygenated cerebrospinal fluid. Then, the oxygenation mechanism 312 supplies the oxygenated cerebrospinal fluid as the high oxygen solution to the injection catheter 52 through the first tube 41. As the oxygenation mechanism 312 in the present modification, a hollow fiber oxygenator for adding oxygen to blood can be used.

The first pump 332 is provided on the path of the first tube 41. The first pump 332 operates based on a control signal transmitted from the control unit 21, and moves liquid from the oxygenation mechanism 312 toward the injection catheter 52.

When the first pump 332 is driven, the first pump 332 supplies a liquid (that is, high oxygen solution) generated in the oxygenation mechanism 312 to the injection catheter 52 through the first tube 41, and finally injects the liquid into the body cavity.

As illustrated in Fig. 23, the reservoir 311 is connected to the discharge catheter 51 via the second tube 42. In addition, the reservoir 311 is connected to the oxygenation mechanism 312 via the third tube 43. The reservoir 311 temporarily stores the cerebrospinal fluid supplied through the second tube 42. Then, the reservoir 311 supplies the stored cerebrospinal fluid to the oxygenation mechanism 312 through the third tube 43. The reservoir 311 has a structure in which the inside and the outside communicate with each other, and can extract gas contained in the stored cerebrospinal fluid to the outside. That is, the reservoir 311 has a function as an air trap.

The second pump 335 is provided on the second tube 42. The second pump 335 operates based on a control signal transmitted from the control unit 21, and moves liquid from the discharge catheter 51 toward the reservoir 311.

When the second pump 335 is driven, the second pump 335 supplies the liquid (that is, cerebrospinal fluid) discharged from the body cavity to the outside of the body cavity through the discharge catheter 51 to the reservoir 311. As a result, the reservoir 311 stores the liquid (that is, cerebrospinal fluid) discharged from the body cavity to the outside of the body cavity through the discharge catheter 51.

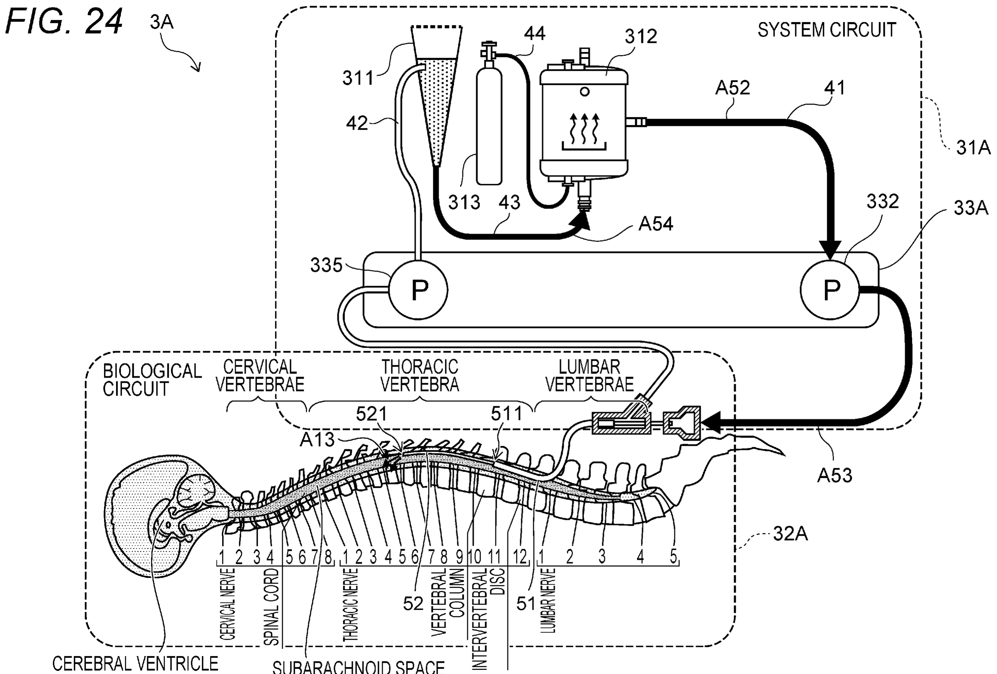

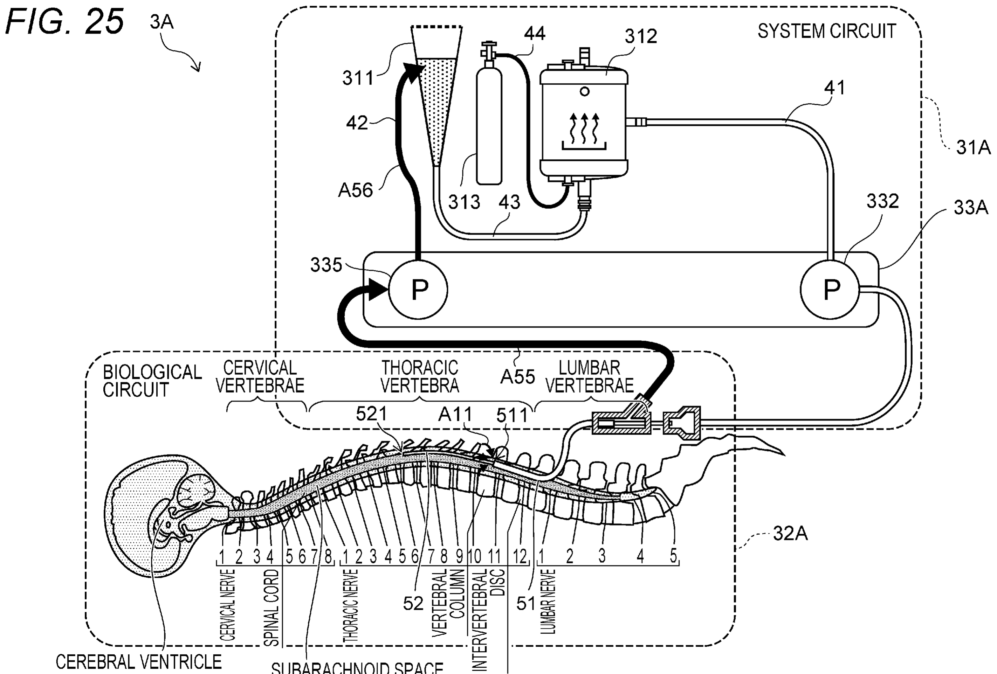

Figs. 24 and 25 are schematic diagrams for describing the operation of the liquid circulation system according to the present modification.

The operation of the liquid circulation system 3A described with reference to Figs. 24 and 25 is the operation based on the control described above with reference to Figs. 8 to 11.

Note that the control unit 21 is omitted in Figs. 24 and 25 for convenience of description. In addition, the following operation will be described on the assumption that the tubes, the reservoir, and the oxygenation mechanism in the circuit start from a primed state filled with artificial cerebrospinal fluid such as lactated Ringer's solution in advance.

First, as illustrated in Fig. 24, as a first step, the control unit 21 transmits a control signal to the first pump 332 to drive the first pump 332. At this time, the control unit 21 brings the second pump 335 into a stationary state.

Then, as indicated by arrows A52 and A53 illustrated in Fig. 24, the liquid 91 (that is, high oxygen solution) generated in the oxygenation mechanism 312 is supplied to the injection catheter 52 through the first tube 41. Then, for example, as indicated by an arrow A13 illustrated in Fig. 24, the liquid 91 is injected into the body cavity from the injection port 521 of the injection catheter 52. Furthermore, as indicated by an arrow A54 illustrated in Fig. 24, the cerebrospinal fluid 92 stored in the reservoir 311 is supplied to the oxygenation mechanism 312 through the third tube 43.

Subsequently, as illustrated in Fig. 25, as a second step, the control unit 21 transmits a control signal to the second pump 335 to drive the second pump 335. At this time, the control unit 21 brings the first pump 332 into a stationary state.

Then, as indicated by an arrow A11 illustrated in Fig. 25, the cerebrospinal fluid 92 in the body cavity is drawn into the discharge port 511 of the discharge catheter 51. Moreover, as indicated by arrows A55 and A56 illustrated in Fig. 25, the cerebrospinal fluid 92 is supplied and stored in the reservoir 311 through the second tube 42.

In the liquid circulation system 3A according to the present modification, a circuit for circulating liquid is defined by being divided into a circuit (that is, biological circuit 32A) including the injection catheter 52 and the discharge catheter 51 and a circuit (that is, system circuit 31A) including a liquid delivery line (that is, first tube 41) connected to the injection catheter 52 and a liquid discharge line (that is, second tube 42) connected to the discharge catheter 51. In addition, the first pump 332 that is provided on the liquid delivery line and moves the liquid and the second pump 335 that is provided on the liquid discharge line and moves the liquid function as a shared pump in the biological circuit 32A and the system circuit 31A. Since the liquid circulation system 3A has such a configuration, the system circuit 31A can cope with an increase in the total volume of the liquid caused by the cavitation phenomenon and a decrease in the total volume of the liquid caused by evaporation while maintaining a situation on a living body side, and can suppress the increase and decrease in the amount of the liquid in the biological circuit. This can suppress fluctuations in intracranial pressure.

In addition, the control unit 21 alternately and repeatedly drives the first pump 332 and the second pump 335. As a result, the injection of the liquid 91 and the discharge of the cerebrospinal fluid 92 are alternately repeated in the same amount as long as an allowable fluctuation range of the intracranial pressure is not exceeded. Therefore, the injection of the liquid 91 and the discharge of the cerebrospinal fluid 92 are not simultaneously performed, and it is possible to suppress the occurrence of locally steady flow in which the liquid 91 injected into the body cavity from the injection port 521 of the injection catheter 52 flows toward the discharge port 511 of the discharge catheter 51. Therefore, the liquid 91 injected into the body cavity flows toward the treatment area of the brain according to the injection direction and the injection flow rate. In addition, the liquid 91 and the cerebrospinal fluid 92 are stirred by the turbulence generated by the resistance between the liquid 91 injected into the body cavity and the cerebrospinal fluid 92 in the body cavity. Furthermore, diffusion due to a concentration difference of oxygen or the like contained in the liquid 91 proceeds. As a result, the liquid can be efficiently delivered to a treatment area of a brain.

In addition, the control unit 21 controls the sum of the injection amount of the liquid ejected while the first pump 332 is continuously or intermittently activated without interposing the activation of the second pump 335 and the discharge amount of the liquid ejected while the second pump 335 is continuously or intermittently activated without interposing the activation of the first pump 332 from the time of the initial activation within a certain range. According to this, it is possible to suppress the fluctuation in the amount of cerebrospinal fluid from the time of initial activation of the first pump 332 and the second pump 335 within a certain range. Therefore, it is possible to more reliably suppress the intracranial pressure from exceeding the allowable fluctuation range. This makes it possible to more reliably suppress the fluctuations in the intracranial pressure within the allowable fluctuation range.

Furthermore, when the injection destination into the body is the subarachnoid space and the liquid to be injected into the subarachnoid space is a high oxygen solution, the high oxygen solution can be efficiently delivered to the treatment area of the brain while suppressing the fluctuation in the intracranial pressure.

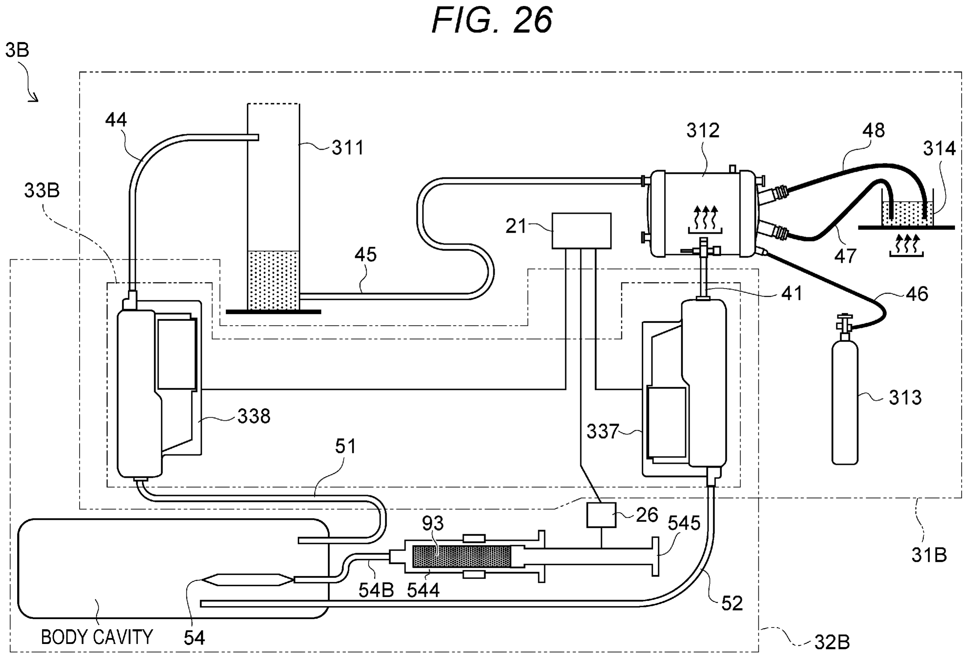

Fig. 26 is a schematic diagram illustrating the outline of the liquid circulation system according to the second modification of the present embodiment.

As illustrated in Fig. 26, the liquid circulation system 3B includes a system circuit 31B, a biological circuit 32B, and a pump unit 33B.

The system circuit 31B is a portion that generates the high oxygen solution, adjusts a temperature, and adjusts a total volume of the liquid in a circulation circuit, and includes the control unit 21, a reservoir 311, an oxygenation mechanism 312, an oxygen supply source 313, and a heat exchanger 314.

The biological circuit 32B is a portion that injects the liquid into the body cavity and discharges the liquid out of the body cavity, and includes the discharge catheter 51, the injection catheter 52, and a balloon catheter 54B. The discharge catheter 51 and the injection catheter 52 are connected to the pump unit 33B and the system circuit 31B including the reservoir 311 and the oxygenation mechanism 312. The discharge catheter 51 and the injection catheter 52 are the same as those described with respect to Figs. 1 to 17. In the liquid circulation system 3B according to the present modification, the injection catheter 52 is not disposed in the lumen 513 (see Fig. 4) of the discharge catheter 51, and exists separately from the discharge catheter 51.

The biological circuit 32B further includes a syringe 544 for a balloon, a piston 545 for a balloon, and a fourth drive unit 26. The syringe 544 for a balloon of the present modification is an example of a "fluid storage unit for balloon" of the present invention, and the syringe 544 for a balloon and the piston 545 for a balloon are an example of a "balloon drive unit" of the present invention. That is, the "balloon drive unit" of the present invention includes the syringe 544 for a balloon and the piston 545 for a balloon. The fourth drive unit 26 of the present modification is an example of a "piston drive unit" of the present invention.

The balloon catheter 54B has a balloon 54 at a distal end portion. When the fourth drive unit 26 moves the piston 545 for a balloon in the insertion direction of the syringe 544 for a balloon based on the control signal transmitted from the control unit 21, the inflation fluid 93 stored in the syringe 544 for a balloon passes through the lumen of the balloon catheter 54B and flows toward the distal end portion of the balloon catheter 54B. Then, the inflation fluid 93 flowing toward the distal end portion of the balloon catheter 54B is supplied to the inside of the balloon 54 through a hole formed in the distal end portion of the balloon catheter 54B. Accordingly, the inflation fluid 93 inflates the balloon 54. That is, the balloon 54 is inflated.

On the other hand, when the fourth drive unit 26 moves the piston 545 for a balloon in the removal direction of the syringe 544 for a balloon based on the control signal transmitted from the control unit 21, the inflation fluid 93 stored in the balloon 54 is drawn and stored in the syringe 544 for a balloon. As a result, the inflation fluid 93 is discharged from the balloon 54, and the balloon 54 deflates. Note that the balloon 54 is desirably designed so as not to strongly press the subarachnoid space near the lumbar vertebrae into which the balloon catheter 54B is inserted even at the time of maximum inflation.

Due to the presence of the spinal cord inside the spinal cavity, the cross-sectional area of the spinal cavity does not become round. In addition, the spine in which the spinal cavity exists gently draws an S-shaped curve as a whole, and may be deformed due to aging, trauma, disease, or the like. Therefore, the balloon 54 is desirably a compliant balloon that is made of an elastic material (stretchable material) such as rubber or elastomer and varies in volume according to the amount of the injected inflation fluid. The compliant balloon may deform to follow the shape of the spinal cavity.

Furthermore, as illustrated in Fig. 26, in the present modification, only one balloon 54 is disposed in the spinal cavity, but the present invention is not limited to this configuration. The balloon 54 may have, for example, a structure in which a plurality of portions to be inflated is provided in the axial direction, and the balloons are connected by a material having flexibility. With this structure, the axial dimension (length) of each balloon can be shortened, and the outer diameter of each balloon at the time of inflation can be limited to a certain value or less. In addition, the internal spaces of the respective balloons may be independent, and since the inflation fluid is injected from a plurality of positions separated in the axial direction, it is possible to uniformly inflate the balloon without changing the total volume at the time of inflation of the entire balloon as compared with the case where the balloon includes only one space. In addition, since the connection portion between the balloon and the balloon becomes an inflection point, it becomes easier to follow the shape of the spinal cavity.

The balloon 54 may also be structured to have multiple independent balloon catheters. Since each balloon catheter can be operated independently, the balloon can be moved and arranged to fill the gap according to the shape and cross-sectional area of the spinal cavity. By implementing this structure, the risk of strongly compressing a part of the spinal cord by the inflation of the balloon 54 is reduced, so that the load on the living body can be further suppressed. That is, the balloon 54 is not particularly limited as long as it follows the shape and cross-sectional area of the spinal cavity and does not strongly press the spinal cord.

The inflation fluid 93 may be a liquid or a gas. Examples of the inflation fluid 93 include physiological saline, cerebrospinal fluid, air, and the like. The inflation fluid 93 may be a fluid different from the liquid injected into the body cavity, or may be a fluid same as the liquid injected into the body cavity.

Examples of the material of the balloon 54 include a stretchable material (that is, compliant material) made of elastomer or rubber such as silicon and natural rubber. However, the material of the balloon 54 is not limited thereto.

The pump unit 33B functions as a liquid delivery unit shared by the system circuit 31B and the biological circuit 32B. The pump unit 33B includes a first pump 337 and a second pump 338. The first pump 337 of the present modification is an example of a "first liquid delivery unit" of the present invention. The second pump 338 of the present modification is an example of a "second liquid delivery unit" of the present invention. Examples of the first pump 337 and the second pump 338 include an infusion pump and a syringe pump.

As illustrated in Fig. 26, the oxygenation mechanism 312 is connected to the first pump 337 via the first tube 41. The first pump 337 is connected to the injection catheter 52. In addition, the oxygenation mechanism 312 is connected to the reservoir 311 via the fifth tube 45. Furthermore, the oxygenation mechanism 312 is connected to the oxygen supply source 313 via the sixth tube 46. The oxygenation mechanism 312 mixes oxygen supplied from the oxygen supply source 313 through the sixth tube 46 with cerebrospinal fluid supplied from the reservoir 311 through the fifth tube 45 or lactated Ringer's solution or a mixed solution of oxygen, cerebrospinal fluid, and an artificial cerebrospinal fluid such as lactated Ringer's solution to generate oxygenated cerebrospinal fluid. The oxygenation mechanism 312 is also connected to the heat exchanger 314 through the seventh tube 47 and the eighth tube 48. The heat exchanger 314 regulates the temperature of the oxygenated cerebrospinal fluid to about 37°C. The temperature adjustment by the heat exchanger is, for example, about 32°C to 40°C. Then, the oxygenation mechanism 312 supplies the oxygenated cerebrospinal fluid as the high oxygen solution to the injection catheter 52 through the first tube 41 and the first pump 337. As the oxygenation mechanism 312 in the present modification, a hollow fiber membrane oxygenator for adding oxygen to blood can be used.

The first pump 337 is connected to the first tube 41 and the injection catheter 52, and is provided between the first tube 41 and the injection catheter 52. The first tube 41 of the present modification is an example of a "liquid delivery line" of the present invention. That is, the first tube 41 is connected to the injection catheter 52 via the first pump 337. The first pump 337 operates based on a control signal transmitted from the control unit 21, and moves liquid from the oxygenation mechanism 312 toward the injection catheter 52.

When the first pump 337 is driven, the first pump 337 supplies the liquid (that is, high oxygen solution) generated in the oxygenation mechanism 312 to the injection catheter 52 through the first tube 41, and finally injects the liquid into the body cavity.

As illustrated in Fig. 26, the reservoir 311 is connected to the second pump 338 via the fourth tube 44. In addition, the reservoir 311 is connected to the oxygenation mechanism 312 via the fifth tube 45. The reservoir 311 temporarily stores the cerebrospinal fluid supplied through the fourth tube 44. Then, the reservoir 311 supplies the stored cerebrospinal fluid to the oxygenation mechanism 312 through the fifth tube 45. The reservoir 311 has a structure in which the inside and the outside communicate with each other, and can extract gas contained in the stored cerebrospinal fluid to the outside. That is, the reservoir 311 has a function as an air trap.

The second pump 338 is connected to the fourth tube 44 and the discharge catheter 51, and is provided between the fourth tube 44 and the discharge catheter 51. The fourth tube 44 of the present modification is an example of a "liquid discharge line" of the present invention. That is, the fourth tube 44 is connected to the discharge catheter 51 via the second pump 338. The second pump 338 operates based on a control signal transmitted from the control unit 21, and moves liquid from the discharge catheter 51 toward the reservoir 311.

When the second pump 338 is driven, the second pump 338 supplies the liquid (that is, cerebrospinal fluid) discharged from the body cavity to the outside of the body cavity through the discharge catheter 51 to the reservoir 311 through the fourth tube 44. As a result, the reservoir 311 stores the liquid (that is, cerebrospinal fluid) discharged from the body cavity to the outside of the body cavity through the discharge catheter 51.

As described in the present modification, the liquid circulation system 3B may include the balloon catheter 54B in which the balloon 54 is provided at the distal end portion. According to the liquid circulation system 3B according to the present modification, the control unit 21 controls the operation of the first pump 337, the operation of the second pump 338, and the inflation and deflation of the balloon 54 disposed in the body cavity, thereby suppressing the fluctuation in the volume of the body cavity as the closed space. As such, the body cavity can vary in volume in a high compliance region and deform to follow the amount of liquid reduced or increased in the body cavity. This suppresses fluctuations in the intracranial pressure. Furthermore, the control unit 21 controls each of the first pump 337 and the second pump 338, and further controls the inflation and deflation of the balloon 54, so that the timing and rate of the injection of the liquid 91 and the deflation of the balloon 54, and the timing and rate of the discharge of the liquid 91 and the inflation of the balloon 54 can be more appropriately set. This further suppresses fluctuations in the intracranial pressure.

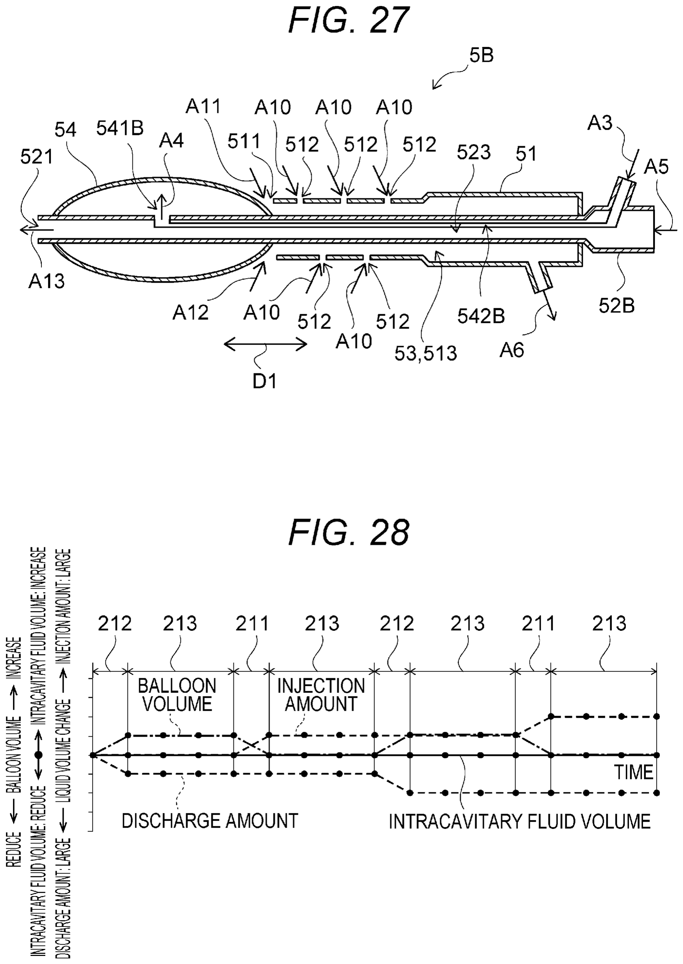

Fig. 27 is a schematic diagram illustrating the modification of the medical device of the present embodiment.

Note that, in a case where the components of the medical device 5B illustrated in Fig. 27 are similar to the components of the medical device 5 described above with reference to Figs. 2 to 4, redundant description will be appropriately omitted, and differences will be mainly described below.

The medical device 5B illustrated in Fig. 27 includes a discharge catheter 51, an injection catheter 52B, and a balloon 54, and is used for the liquid circulation system 3B described above with reference to Fig. 26, for example. The balloon 54 is provided at a distal end portion of the injection catheter 52B. That is, the injection catheter 52B has the balloon 54 at the distal end portion. The discharge catheter 51 is as previously described with respect to Figs. 2 to 4.

The injection catheter 52B is positioned in the lumen 513 of the discharge catheter 51 and is not coupled with the discharge catheter 51. Therefore, the injection catheter 52B is movable in the lumen 513 of the discharge catheter 51 along the longitudinal direction D1 of the discharge catheter 51. Since the distal end portion of the discharge catheter 51 is opened as the discharge port 511, as illustrated in Fig. 27, the distal end portion of the injection catheter 52B (that is, the portion provided with the balloon 54) can pass through the discharge port 511 of the discharge catheter 51. That is, the medical device 5B has a structure in which only the injection catheter 52B and the balloon 54 are integrally formed and are not coupled to the discharge catheter 51. By adopting this structure, it is possible to change the positions of the injection catheter and the discharge catheter, so that it is possible to position the injection catheter according to the shape of the patient's spinal cavity.

Note that the balloon 54 of the present embodiment is not limited to being formed integrally with the injection catheter. For example, as in the liquid circulation system 3B described above with respect to Fig. 26, the balloon 54 may be formed separately from the injection catheter and provided at the distal end portion of the balloon catheter 54B.

In addition, in the present modification, the distance in the longitudinal direction D1 between the distal end portion of the discharge catheter 51 and the distal end portion of the injection catheter 52B exposed from the discharge port 511 of the discharge catheter 51 is preferably, for example, about 0 mm or more and 300 mm or less. In addition, the cross-sectional area of the balloon 54 is preferably about half of the cross-sectional area of the spinal cavity, for example, about 3.3 mm² to 8.3 mm², considering that the cross-sectional area of the general spinal cavity is about 6.7 mm² to 16.7 mm² and the discharge catheter 51 is disposed in the spinal cavity separately from the balloon 54. The length of the balloon 54 along the longitudinal direction D1 is, for example, about 1 mm to 300 mm, preferably 36 mm to 200 mm. As a result, by arranging the injection catheter 52B at a position separated from the distal end of the discharge catheter 51 by the length of the balloon 54, when the balloon 54 is inflated, the balloon 54 and the discharge port 511 at the distal end portion of the discharge catheter 51 can function without coming into contact with each other and interfering with each other.

For example, as indicated by an arrow A3 illustrated in Fig. 27, the inflation fluid supplied from the proximal end portion of the injection catheter 52B passes through a lumen 542B formed inside the injection catheter 52B and flows toward the distal end portion of the injection catheter 52B. Then, for example, as indicated by an arrow A4 illustrated in Fig. 27, the inflation fluid flowing toward the distal end portion of the injection catheter 52B is supplied to the inside of the balloon 54 through a hole 541B formed at the distal end portion of the injection catheter 52B. Accordingly, the inflation fluid inflates the balloon 54. That is, the balloon 54 is inflated. On the other hand, when the inflation fluid is discharged from the balloon 54, the balloon 54 is deflated.

For example, as indicated by arrows A11 and A12 shown in Fig. 27, the discharge catheter 51 draws cerebrospinal fluid present in the subarachnoid space near the lumbar vertebrae into the space 53 through the discharge port 511. In addition, for example, as indicated by an arrow A10 illustrated in Fig. 27, the discharge catheter 51 draws cerebrospinal fluid existing in the subarachnoid space near the lumbar vertebrae into the space 53 through the plurality of holes 512. Then, as indicated by the arrow A6 illustrated in Fig. 27, the discharge catheter 51 discharges the cerebrospinal fluid out of the body cavity of the subject through the space 53.

For example, as indicated by an arrow A5 illustrated in Fig. 27, the liquid supplied from the proximal end portion of the injection catheter 52B flows toward the distal end portion of the injection catheter 52B through the lumen 523 of the injection catheter 52B. Then, for example, as indicated by an arrow A13 illustrated in Fig. 27, the injection catheter 52B injects the liquid from the injection port 521 into the cerebrospinal fluid existing in the subarachnoid space through the lumen 523 of the injection catheter 52B.

Hereinafter, details of control of the medical system according to a modification of the present embodiment will be described with reference to the drawings.

Fig. 28 is a graph showing a relationship among an injection amount, a discharge amount, a balloon volume, and an intracavitary fluid volume by control of the medical system according to the second modification of the present embodiment.

In the description regarding the control of the present modification, a case where the medical device 5B described above with reference to Fig. 27 is used will be described as an example.

The horizontal axis of the graph shown in Fig. 28 represents time. The vertical axis of the graph shown in Fig. 28 represents the transition of the liquid amount. Specifically, the "injection amount" illustrated in Fig. 28 represents an integrated amount of the liquid 91 injected into the body cavity from the start of execution of the control. The "discharge amount" illustrated in Fig. 28 represents an integrated amount of the cerebrospinal fluid discharged out of the body cavity from the start of execution of the control. The "intracavitary fluid volume" illustrated in Fig. 28 represents a change amount of the cerebrospinal fluid present in the body cavity at the start of execution of the control. In addition, the vertical axis of the graph illustrated in Fig. 28 represents a change in the volume of the balloon 54.

As illustrated in Fig. 28, in the control of the present modification, the control unit 21 sets a second period 212 in which the liquid 91 is not injected into the body cavity, the liquid 91 is discharged from the body cavity, and the balloon 54 disposed in the body cavity is inflated, and a first period 211 in which the liquid 91 is injected into the body cavity, the liquid 91 is not discharged from the body cavity, and the balloon 54 is deflated.

As indicated by arrows A10 and A6 illustrated in Fig. 27, in the second period 212, the discharge catheter 51 draws the cerebrospinal fluid 92 (including liquid 91) existing in the subarachnoid space near the lumbar vertebrae into the space 53 through the plurality of holes 512 and discharges the cerebrospinal fluid out of the subject body cavity through the space 53. In addition, as indicated by arrows A3 and A4 illustrated in Fig. 27, in the second period 212, the inflation fluid 93 is supplied from the proximal end portion of the medical device 5B, passes through the lumen 542B, passes through the hole 541B, and is supplied to the inside of the balloon 54. As a result, the balloon 54 is inflated.

According to this, in the second period 212 in which the cerebrospinal fluid 92 (including liquid 91) is discharged from the body cavity, the control unit 21 can suppress the fluctuation in the volume of the body cavity as the closed space. As such, the body cavity can vary in volume in a high compliance region and deform to follow the amount of liquid reduced in the body cavity. Therefore, the fluctuation in the intracranial pressure in the second period 212 is suppressed.

In the second period 212, the control unit 21 simultaneously discharges the cerebrospinal fluid 92 (including liquid 91) and inflates the balloon 54. In addition, in the second period 212, the control unit 21 sets the inflation amount of the balloon 54 (that is, injection amount of inflation fluid 93 into balloon 54) to be substantially the same as the discharge amount of the cerebrospinal fluid 92 (including liquid 91).

According to this, the amount (that is, volume) of liquid reduced in the body cavity is substantially the same as the amount (that is, volume) of the balloon 54 inflated in the body cavity. Therefore, the volume of the body cavity does not substantially change in the second period 212. As a result, the fluctuation in the intracranial pressure in the second period 212 is more reliably suppressed.

On the other hand, as indicated by arrows A5 and A13 illustrated in Fig. 27, in the first period 211, the injection catheter 52B injects the liquid 91 supplied from the proximal end portion of the injection catheter 52B into the cerebrospinal fluid existing in the subarachnoid space through the lumen 523 from the injection port 521. In the first period 211, the inflation fluid 93 inside the balloon 54 passes through the hole 541B, passes through the lumen 542B, and is discharged to the outside of the balloon 54. As a result, the balloon 54 deflates.

According to this, the control unit 21 can suppress the fluctuation in the volume of the body cavity as the closed space in the first period 211 in which the liquid 91 is injected into the body cavity. As such, the body cavity can vary in volume in a high compliance region and deform to follow the increased amount of liquid in the body cavity. Therefore, the fluctuation in the intracranial pressure in the first period 211 is suppressed.

In the first period 211, the control unit 21 simultaneously executes injection of the liquid 91 and deflation of the balloon 54. In addition, in the first period 211, the control unit 21 sets the deflation amount of the balloon 54 (that is, discharge amount of inflation fluid 93 from balloon 54) to be substantially the same as the injection amount of the liquid 91.

According to this, the amount (that is, volume) of liquid increased in the body cavity is substantially the same as the amount (that is, volume) of the balloon 54 deflated in the body cavity. Therefore, the volume of the body cavity does not substantially change in the first period 211. As a result, the fluctuation in the intracranial pressure in the first period 211 is more reliably suppressed.

Then, as illustrated in Fig. 28, the control unit 21 alternately repeats the second period 212 and the first period 211. In the control of the medical system according to the present modification, the injection and discharge of the liquid 91 are not simultaneously performed, and it is possible to suppress the generation of the locally steady flow in which the liquid 91 injected into the body cavity from the injection port 521 of the injection catheter 52B flows toward the discharge port 511 of the discharge catheter 51.

As illustrated in Fig. 28, the control unit 21 further sets a third period 213 in which the liquid 91 is not injected into the body cavity and the liquid 91 is not discharged from the body cavity between the second period 212 and the first period 211 and between the first period 211 and the second period 212. That is, in the third period 213, the injection of the liquid 91 into the body cavity and the discharge of the liquid 91 from the body cavity are simultaneously stopped. The control unit 21 repeats the second period 212, the third period 213, the first period 211, and the third period 213 in this order. In the third period, the diffusion due to the concentration difference of oxygen or the like contained in the liquid 91 progresses toward the brain side and the lumbar side, for example, as indicated by arrows A24 and A25 in Fig. 10, according to the duration of the third period 213 (that is, stop time). The duration of the third period 213 is preferably until the flow generated by the immediately preceding injection or discharge disappears, and specifically, 1 second or more is preferable.

The control unit 21 executes control to maintain the balloon 54 in the inflated state in the third period 213 between the second period 212 and the first period 211. For example, in the third period 213 between the second period 212 and the first period 211, the control unit 21 maintains the balloon 54 in the inflated state by maintaining the state in which the inflation fluid 93 is supplied to the inside of the balloon 54.

On the other hand, the control unit 21 executes control to maintain the balloon 54 in the deflated state in the third period between the first period and the second period. For example, in the third period between the first period and the second period, the control unit 21 maintains the balloon 54 in a deflated state by maintaining a state in which the inflation fluid 93 is discharged to the outside of the balloon 54.

As described above, according to the liquid circulation system 3B according to the second modification and the medical system according to the present modification, the amount (that is, volume) of the liquid increased or decreased in the body cavity is substantially the same as the amount (that is, volume) of the balloon 54 increased or decreased in the body cavity. Therefore, the volume of the body cavity does not substantially change. This suppresses fluctuations in the intracranial pressure. In addition, since the volume of the body cavity does not substantially change, the body cavity can vary in volume in the high compliance region regardless of the injection amount of the liquid 91 and deform following the increased or decreased amount of liquid in the body cavity. Therefore, even in a case where the balance of the amount of the liquid is somewhat disturbed, the body cavity can accommodate the imbalance due to its compliance. Further, because the volume of the body cavity does not substantially change regardless of the injection amount of the liquid 91, the injection amount of the liquid 91 can be increased as long as the inflatable space of the balloon 54 is allowed.

On the other hand, since the volume of the balloon 54 cannot be a negative amount, it is necessary to adjust the balance of the integration of the injection amount and the discharge amount of the fluid with respect to the balloon 54 after the balloon 54 is deflated in the first period 211 so as not to be negative at any timing. Specifically, the injection amount into the balloon and the discharge amount from the balloon are limited to "(sum of n balloon injections) - (sum of n-1 balloon discharges) ≥ n-th discharge". The discharge amount per one time is not particularly limited as long as it does not exceed the volume of the spinal cavity, and is, for example, about 1 mL to 30 mL. The injection amount per one time is about 1 mL to 30 mL, and more preferably about 3 to 10 mL considering the cross-sectional area of the balloon 54 and the length that can be disposed along the longitudinal direction D1.

Furthermore, as described above, the control unit 21 further sets the third period 213 in which the liquid 91 is not injected into the body cavity and the liquid 91 is not discharged from the body cavity between the second period 212 and the first period 211 and between the first period 211 and the second period 212. Therefore, the diffusion of oxygen and the like contained in the liquid 91 further proceeds according to the length of the third period. As a result, the liquid can be more efficiently delivered to a treatment area of a brain.

The embodiment of the present invention has been described above. However, the present invention is not limited to the embodiments, and various modifications can be made without departing from the scope of the claims. The configurations of the embodiments may be partially omitted or optionally combined to make different configurations from the aforementioned configurations.

### Reference Signs List

- 2: Medical system
- 3: Liquid circulation system
- 3A: Liquid circulation system
- 3B: Liquid circulation system
- 5: Medical device
- 5B: Medical device
- 21: Control unit
- 22: Pump
- 23: First drive unit
- 24: Second drive unit
- 26: Fourth drive unit
- 31: System circuit
- 31A: System circuit
- 31B: System circuit
- 32: Biological circuit
- 32A: Biological circuit
- 32B: Biological circuit
- 33: Pump unit
- 33A: Pump unit
- 33B: Pump unit
- 41: First tube
- 42: Second tube
- 43: Third tube
- 44: Fourth tube
- 45: Fifth tube
- 46: Sixth tube
- 47: Seventh tube
- 48: Eighth tube
- 51: Discharge catheter
- 52: Injection catheter
- 52B: Injection catheter
- 53: Space
- 54: Balloon
- 54B: Balloon catheter
- 55: Tube
- 56: Tank
- 91: Fluid
- 92: Cerebrospinal fluid
- 93: Inflation fluid
- 211: First period
- 211a: First period
- 212: Second period
- 212a: Second period
- 213: Third period
- 311: Reservoir
- 312: Oxygenation mechanism
- 313: Oxygen supply source
- 314: Heat exchanger
- 331: First liquid storage unit
- 332: First pump
- 333: First flow path switching unit
- 334: Second liquid storage unit
- 335: Second pump
- 336: Second flow path switching unit
- 337: First pump
- 338: Second pump
- 511: Discharge port
- 512: Hole
- 513: Lumen
- 521: Injection port
- 523: Lumen
- 541B: Hole
- 542B: Lumen
- 544: Syringe for balloon
- 545: Piston for balloon
- 911: Colored water

## Claims

1. A medical system that injects a liquid into a body cavity in which cerebrospinal fluid of a subject is present and discharges the liquid present in the body cavity from the body cavity, the medical system comprising
a control unit that executes control of setting
a first period in which the liquid is injected into the body cavity and the liquid is not discharged from the body cavity and
a second period in which the liquid is not injected into the body cavity and the liquid is discharged from the body cavity, and alternately repeating the first period and the second period to set an injection amount of the liquid in the first period to be substantially the same as a discharge amount of the liquid in the second period.

2. The medical system according to claim 1, wherein a cycle from a start of the first period to an end of the second period is substantially constant.

3. The medical system according to claim 1, wherein the control unit further sets, between the first period and the second period, a third period in which the liquid is not injected into the body cavity and the liquid is not discharged from the body cavity.

4. The medical system according to claim 3, wherein the duration of the third period is longer than the duration of the first period.

5. The medical system according to claim 3, wherein the duration of the third period is 1 second or more.

6. The medical system according to claim 3, wherein
the control unit further sets the third period between the second period and the first period, and
a first time of the third period set between the first period and the second period is longer than a second time of the third period set between the second period and the first period.

7. The medical system according to claim 1, wherein a discharge rate of the liquid in the second period is slower than an injection rate of the liquid in the first period.

8. The medical system according to claim 1, wherein
the control unit sets a 0th period for injecting or discharging an amount of the liquid smaller than the injection amount in the first period and the discharge amount in the second period as an initial period, and
starts a period, among the first period and the second period, in which a moving direction of the liquid is different from that in the 0th period after the 0th period.

9. The medical system according to claim 8, wherein an injection amount in the 0th period is substantially half of the injection amount in the first period, and a discharge amount in the 0th period is substantially half of the discharge amount in the second period.

10. The medical system according to claim 1, wherein at the end of the control, the control unit performs control to cause an amount of the cerebrospinal fluid to approach an amount of the cerebrospinal fluid existing in the body cavity at the start of execution of the control by injecting the liquid into the body cavity or to cause an amount of the cerebrospinal fluid to approach an amount of the cerebrospinal fluid existing in the body cavity at the start of execution of the control by discharging the liquid from the body cavity.

11. The medical system according to claim 1, further comprising:
an injection catheter that injects the liquid into the body cavity; and
a discharge catheter that discharges the liquid from the body cavity, wherein
a distance between a distal end of the injection catheter and a distal end of the discharge catheter is 30 cm or less at a start of execution of the control.

12. The medical system according to claim 1, wherein the control unit further executes control to deflate a balloon disposed in the body cavity and capable of inflation and deflation in the first period and to inflate the balloon in the second period.

13. The medical system according to claim 12, wherein the control unit simultaneously executes injection of the liquid and the deflation of the balloon in the first period, and simultaneously executes discharge of the liquid and the inflation of the balloon in the second period.

14. The medical system according to claim 12, wherein the control unit sets a deflation amount of the balloon to be substantially the same as an injection amount of the liquid in the first period, and sets an inflation amount of the balloon to be substantially the same as a discharge amount of the liquid in the second period.

15. The medical system according to claim 12, wherein the control unit further sets a third period in which the liquid is not injected into the body cavity and the liquid is not discharged from the body cavity between the first period and the second period and between the second period and the first period, maintains the balloon in the deflated state in the third period between the first period and the second period, and maintains the balloon in the inflated state in the third period between the second period and the first period.

16. A liquid circulation system that circulates a liquid by injecting the liquid into a body cavity in which cerebrospinal fluid is present and discharging the liquid to the outside of the body cavity, the liquid circulation system comprising:
an injection catheter that injects the liquid into the body cavity;
a discharge catheter that discharges the liquid from the body cavity to the outside of the body cavity;
a liquid delivery line connected to the injection catheter;
a liquid discharge line connected to the discharge catheter;
a first liquid delivery unit that is provided on the liquid delivery line and moves the liquid;
a second liquid delivery unit that is provided on the liquid discharge line and moves the liquid; and
a single control unit that controls the first liquid delivery unit and the second liquid delivery unit.

17. The liquid circulation system according to claim 16, further comprising:
a liquid storage unit that stores at least one of the liquid to be injected into the body cavity and the liquid discharged from the body cavity; and
a liquid treatment device provided between the liquid storage unit in the liquid delivery line and the first liquid delivery unit to treat the liquid.

18. The liquid circulation system according to claim 17, wherein the liquid treatment device is an oxygenator that adds oxygen to the liquid discharged out of the body cavity.

19. The liquid circulation system according to claim 16, wherein the control unit alternately and repeatedly drives the first liquid delivery unit and the second liquid delivery unit.

20. The liquid circulation system according to claim 16, wherein the control unit controls a sum of an injection amount of the liquid ejected while the first liquid delivery unit is continuously or intermittently activated without interposing the activation of the second liquid delivery unit and a discharge amount of the liquid ejected while the second liquid delivery unit is continuously or intermittently activated without interposing the activation of the first liquid delivery unit from a time of initial activation within a certain range.

21. The liquid circulation system according to claim 16, further comprising
a balloon disposed in the body cavity and capable of inflation and deflation, wherein
the control unit further controls the inflation and the deflation of the balloon.

22. The liquid circulation system according to claim 21, further comprising
a balloon drive unit that includes a fluid storage unit for a balloon that stores an inflation fluid for inflating the balloon, and performs an operation of injecting the inflation fluid in the fluid storage unit for a balloon into the balloon and an operation of sending the inflation fluid from the inside of the balloon into the fluid storage unit for a balloon, wherein
the balloon drive unit injects the inflation fluid into the balloon to inflate the balloon, and discharges the inflation fluid from the balloon to deflate the balloon.
